# EUROPÄISCHE PATENTSCHRIFT

(11) **EP 0 769 010 B1**
(45) Veröffentlichungstag und Bekanntmachung des Hinweises auf die Patenterteilung: **30.08.2000**
(21) Anmeldenummer: 95924301.5
(22) Anmeldetag: 21.06.1995
(51) Int. Cl.: C07D 249/12, C07D 401/04, C07D 403/04, A01N 43/653, A01N 47/08, C07C 239/08, C07C 271/06

(54) **2-[1',2',4'-TRIAZOL-3'YLOXYMETHYLEN]-ANILIDE UND IHRE VERWENDUNG ALS SCHÄDLINGSBEKÄMPFUNGSMITTEL**
2-[1',2',4'-TRIAZOL-3'-YLOXYMETHYLENE]-ANILIDES AND THEIR USE AS PEST-CONTROL AGENTS
2-[1',2',4'-TRIAZOL-3'-YLOXYMETHYLENE]-ANILIDES ET LEUR UTILISATION COMME PESTICIDES

(30) Priorität: 06.07.1994 DE 4423613
(43) Veröffentlichungstag der Anmeldung: 23.04.1997
(73) Patentinhaber: BASF AKTIENGESELLSCHAFT, 67056 Ludwigshafen (DE)
(72) Erfinder: MÜLLER, Bernd, D-67227 Frankenthal (DE); SAUTER, Hubert, D-68167 Mannheim (DE); GÖTZ, Norbert, D-67547 Worms (DE); KÖNIG, Hartmann, D-69115 Heidelberg (DE); RÖHL, Franz, D-67105 Schifferstadt (DE); LORENZ, Gisela, D-67434 Hambach (DE); AMMERMANN, Eberhard, D-64646 Heppenheim (DE)
(86) Internationale Anmeldenummer: EP9502395
(87) Internationale Veröffentlichungsnummer: WO9601258

(56) Entgegenhaltungen:
- EP-A- 0 525 516
- EP-A- 0 619 301
- WO-A-93/15046
- US-A- 3 547 977
- DATABASE CROSSFIRE Beilstein Informationssysteme GmbH, Frankfurt DE & CHEM. BER., Bd. 25, 1892 Seite 2445
- DATABASE CROSSFIRE Beilstein Informationssysteme GmbH, Frankfurt DE & J. MED. CHEM., Bd. 34, Nr. 9, 1991 Seiten 2906-2916,
- DATABASE CROSSFIRE Beilstein Informationssysteme GmbH, Frankfurt DE & CHEM. BER., Bd. 37, 1904 Seite 3599
- DATABASE CROSSFIRE Beilstein Informationssysteme GmbH, Frankfurt DE & J. AMER. CHEM. SOC., Bd. 80, 1958 Seite 1168, 1171
- DATABASE CROSSFIRE Beilstein Informationssysteme GmbH, Frankfurt DE & CH,A,558 783 (LILLY) 1975
- DATABASE CROSSFIRE Beistein Informationssysteme GmbH, Frankfurt DE & CHEM. BER., Bd. 27, 1894 Seite 2162
- DATABASE CROSSFIRE Beilstein Informationssysteme GmbH, Frankfurt DE & HELV. CHIM. ACTA, Bd. 63, Nr. 8, 1980 Seiten 2364-2369,
- DATABASE CROSSFIRE Beilstein Informationssyteme GmbH, Frankfurt DE & JUSTUS LIEBIGS ANN. CHEM., Bd. 316, 1901 Seite 278, 295, 287, 289
- DATABASE CROSSFIRE Beilstein Informationssysteme GmbH, Frankfurt DE & BULL. SOC. CHIM. FR., 1966 Seiten 1848-1858,
- DATABASE CROSSFIRE Beilstein Informationssysteme GmbH, Frankfurt DE & CHEM. BER., Bd. 40, 1907 Seite 3330
- DATABASE CROSSFIRE Beilstein Informationssysteme GmbH, Frankfurt DE & US,A,2 334 201 (PARKE, DAVIS & CO.) 1941

## Beschreibung

Die vorliegende Erfindung betrifft 2-[1',2',4'-Triazol-3'-yloxymethylen]-anilide der Formel I 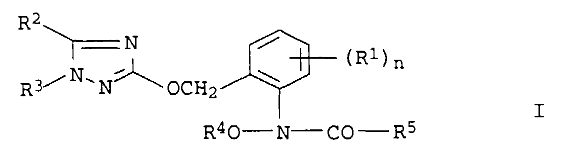 in der der Index und die Substituenten die folgende Bedeutung haben:
- n: 0, 1, 2, 3 oder 4, wobei die Substituenten R¹ verschieden sein können, wenn n größer als 1 ist;
- R¹: Halogen, C₁-C₄-Alkyl, C₁-C₂-Halogenalkyl, C₁-C₄-Alkoxy oder C₁-C₂-Halogenalkoxy;
- R²: Wasserstoff, Nitro, Cyano, Halogen, C₁-C₄-Alkyl, C₁-C₄-Halogenalkyl, C₁-C₄-Alkoxy, C₁-C₄-Alkylthio oder C₁-C₄-Alkoxycarbonyl;
- R³: C₁-C₄-Alkyl oder C₃-C₆-Cycloalkyl
Phenyl oder Benzyl, wobei der Phenylteil partiell oder vollständig halogeniert sein kann und/oder
- ein bis drei der folgenden Reste: Cyano, Nitro, C₁-C₆-Alkyl, C₁-C₄-Halogenalkyl, C₁-C₄-Alkoxy, C₁-C₄-Halogenalkoxy, C₁-C₄-Alkoxy-C₁-C₄-alkyl, C₃-C₆-Cycloalkyl, C₁-C₄-Alkylcarbonyl, C₁-C₄-Alkoxycarbonyl, Phenyl, Phenoxy und Phenyl-C₁-C₄-alkoxy, wobei die Phenylringe ihrerseits partiell oder vollständig halogeniert sein können und/oder ein bis drei der folgenden Reste tragen können: Cyano, Nitro, C₁-C₄-Alkyl, C₁-C₂-Halogenalkyl, C₁-C₄-Alkoxy, C₁-C₂-Halogenalkoxy, C₃-C₆-Cycloalkyl, C₁-C₄-Alkylcarbonyl oder C₁-C₄-Alkoxycarbonyl, und/oder
- eine Gruppe CR'=NOR", in der R' Wasserstoff oder C₁-C₄-Alkyl bedeutet und R" für C₁-C₆-Alkyl steht, und/oder
- zwei benachbarte C-Atome des Phenylrings über eine Oxy-C₁-C₃-alkoxy-Brücke oder eine Oxy-C₁-C₃-halogenalkoxy-Brücke
tragen kann,
Pyridyl oder Pyrimidyl, wobei der heteroaromatische Ring partiell oder vollständig halogeniert sein kann und/oder ein bis drei der folgenden Reste tragen kann: Cyano, Nitro, C₁-C₄-Alkyl, C₁-C₂-Halogenalkyl, C₁-C₄-Alkoxy, C₁-C₂-Halogenalkoxy, C₃-C₆-Cycloalkyl, C₁-C₄-Alkylcarbonyl oder C₁-C₄-Alkoxycarbonyl;
- R⁴: Wasserstoff, C₁-C₄-Alkyl oder C₁-C₂-Halogenalkyl;
- R⁵: Methyl, Ethyl, Cyclopropyl, Methoxy oder Methylamino.

Außerdem betrifft die Erfindung Verfahren und Zwischenprodukte zur Herstellung dieser Verbindungen, sie enthaltende Mittel sowie ihre Verwendung zur Bekämpfung von tierischen Schädlingen oder Schadpilzen.

EP-A 325 516 beschreibt Zimtsäurederivate mit fungizider und insektizider Wirkung, wobei der Phenylring der Zimtsäure einen Aryloxymethylen- bzw. einen 1,2,4-Triazol-5-yl-thiomethylen-Substituenten in 2-Stellung tragen kann.

Aus der WO-A 93/15,046 sind 2-[1,2,4-Triazol-5-yloxymethylen]-anilide zur Bekämpfung von Schadpilzen bekannt.

Der vorliegenden Erfindung lagen Verbindungen mit verbesserter Wirkung als Aufgabe zugrunde.

Demgemäß wurden die eingangs definierten Verbindungen I gefunden. Des weiteren wurden Verfahren und Zwischenprodukte zu ihrer Herstellung, sie enthaltende Mischungen sowie Verfahren zur Bekämpfung von Insekten, Spinnentieren und Nematoden und Schadpilzen unter Verwendung der Verbindungen I gefunden.

Die Verbindungen I sind auf verschiedenen Wegen erhältlich.

Man erhält diejenigen Verbindungen I, in denen R⁴ Wasserstoff bedeutet und R⁵ für Methyl, Ethyl, Cyclopropyl oder Methoxy steht, beispielsweise dadurch, daß man ein Benzylderivat der Formel II in Gegenwart einer Base mit einem 3-Hydroxytriazol der Formel III in das entsprechende 2-[1,2,4-Triazol-3-yloxymethylen]-nitrobenzol der Formel IV überführt, IV anschließend zum N-Hydroxylanilin der Formel Va reduziert und Va mit einer Carbonylverbindung der Formel VI in I umwandelt.

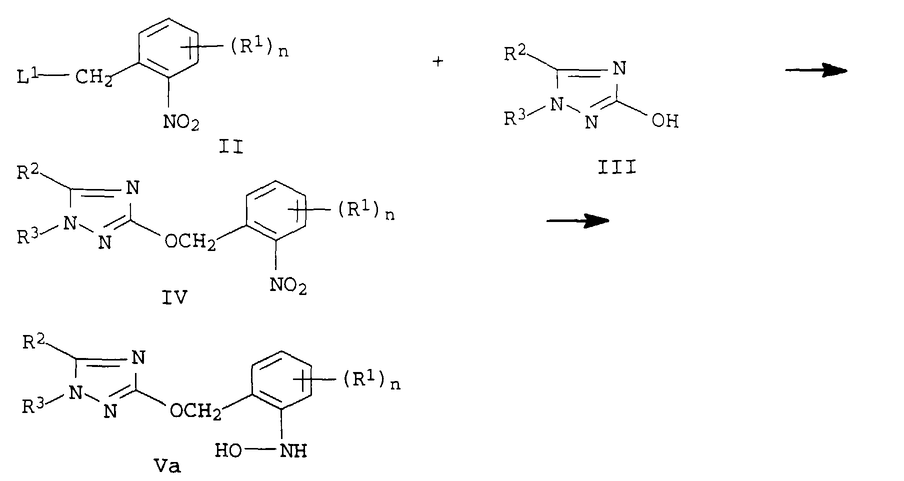

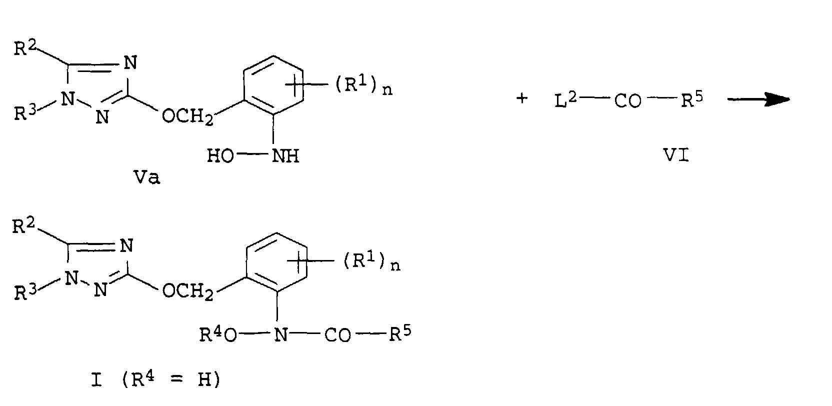

L¹ in der Formel II und L² in der Formel VI bedeuten jeweils eine nucleophil austauschbare Gruppe, beispielsweise Halogen (z.B. Chlor, Brom und Iod), oder ein Alkyl- oder Arylsulfonat (z.B. Methylsulfonat, Trifluormethylsulfonat, Phenylsulfonat und 4-Methylphenylsulfonat).

Die Veretherung der Verbindungen II und III wird üblicherweise bei Temperaturen von 0°C bis 80°C, vorzugsweise 20°C bis 60°C, durchgeführt.

Geeignete Lösungsmittel sind aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Alkohole wie Methanol, Ethanol, n-Propanol, i-Propanol, n-Butanol und tert.-Butanol, Ketone wie Aceton und Methylethylketon sowie Dimethylsulfoxid, Dimethylformamid, Dimethylacetamid, 1,3-Dimethylimidazolidin-2-on und 1,2-Dimethyltetrahydro-2(1H)-pyrimidin, vorzugsweise Methylenchlorid, Aceton, Toluol, tert.-Butylmethylether und Dimethylformamid. Es können auch Gemische der genannten Lösungsmittel verwendet werden.

Als Basen kommen allgemein anorganische Verbindungen wie Alkalimetall- und Erdalkalimetallhydroxide (z.B. Lithiumhydroxid, Natriumhydroxid, Kaliumhydroxid und Calziumhydroxid), Alkalimetall- und Erdalkalimetalloxide (z.B. Lithiumoxid, Natriumoxid, Calziumoxid und Magnesiumoxid), Alkalimetall- und Erdalkalimetallhydride (z.B. Lithiumhydrid, Natriumhydrid, Kaliumhydrid und Calziumhydrid), Alkalimetallamide (z.B. Lithiumamid, Natriumamid und Kaliumamid), Alkalimetall- und Erdalkalimetallcarbonate (z.B. Lithiumcarbonat und Calziumcarbonat) sowie Alkalimetallhydrogencarbonate (z.B. Natriumhydrogencarbonat), metallorganische Verbindungen, insbesondere Alkalimetallalkyle (z.B. wie Methyllithium, Butyllithium und Phenyllithium), Alkylmagnesiumhalogenide (z.B. Methylmagnesiumchlorid) sowie Alkalimetall- und Erdalkalimetallalkoholate (z.B. Natriummethanolat, Natriumethanolat, Kaliumethanolat, Kalium-tert.-Butanolat und Dimethoxymagnesium), außerdem organische Basen, z.B. tertiäre Amine wie Trimethylamin, Triethylamin, Tri-isopropylethylamin und N-Methylpiperidin, Pyridin, substituierte Pyridine wie Collidin, Lutidin und 4-Dimethylaminopyridin sowie bicyclische Amine in Betracht.

Besonders bevorzugt werden Natriumhydroxid, Kaliumcarbonat und Kalium-tert.-butanolat.

Die Basen werden im allgemeinen äquimolar, im Überschuß oder gegebenenfalls als Lösungsmittel verwendet.

Es kann für die Umsetzung vorteilhaft sein, eine katalytische Menge eines Kronenethers (z.B. 18-Krone-6 oder 15-Krone-5) zuzusetzen.

Die Umsetzung kann auch in Zweiphasensystemen bestehend aus einer Lösung von Alkali- oder Erdalkalihydroxiden oder -carbonaten in Wasser und einer organischen Phase (z.B. aromatische und/oder halogenierte Kohlenwasserstoffe) durchgeführt werden. Als Phasentransferkatalysatoren kommen hierbei beispielsweise Ammoniumhalogenide und -tetrafluoroborate (z.B. Benzyltriethylammoniumchlorid, Benzyltributylammoniumbromid, Tetrabutylammoniumchlorid, Hexadecyltrimethylammoniumbromid oder Terabutylammoniumtetrafluoroborat) sowie Phosphoniumhalogenide (z.B. Tetrabutylphosphoniumchlorid und Tetraphenylphosphoniumbromid) in Betracht.

Es kann für die Umsetzung vorteilhaft sein, zunächst das 3-Hydroxytriazol mit der Base in das entsprechende Hydroxylat umzusetzen, welches dann mit dem Benzylderivat umgesetzt wird.

Die für die Herstellung der Verbindungen I benötigten Ausgangsstoffe II sind aus EP-A 513 580 bekannt oder können nach den dort beschriebenen Methoden hergestellt werden [Synthesis 1991, 181; Anal. Chim. Acta 185, 295 (1986); EP-A 336 567].

3-Hydroxytriazole III sind ebenfalls aus der Literatur bekannt oder können nach den dort beschriebenen Methoden hergestellt werden [Chem. Ber. 56, 1794 (1923); DE-A 21 50 169; DE-A 22 00 436; US-A 4,433,148; J. Med. Chem. 33, 2772 (1990); Synthesis 1987, 986; DE-A 22 60 015; DE-A 24 17 970].

Die Reduktion der Nitroverbindungen IV zu den entsprechenden N-Hydroxyanilinen IVa erfolgt analog zu literaturbekannten Methoden beispielsweise mit Metallen wie Zink [vgl. Ann. Chem. 316, 278 (1901)] oder mit Wasserstoff (vgl. EP-A 085 890).

Die Umsetzung der N-Hydroxyaniline Va mit den Carbonylverbindungen VI erfolgt unter alkalischen Bedingungen insbesondere bei Temperaturen von -10°C bis 30°C. Die bevorzugten Lösungsmittel sind Methylenchlorid, Toluol, tert.-Butylmethylether oder Essigsäureethylester. Die bevorzugten Basen sind Natriumhydrogencarbonat, Kaliumcarbonat, Natriumhydroxid oder wäßrige Natriumhydroxid-Lösung.

Außerdem erhält man die Verbindungen der Formel I, in denen R⁴ nicht Wasserstoff bedeutet und R⁵ für Methyl, Ethyl, Cyclopropyl oder Methoxy steht, beispielsweise dadurch, daß man eine Verbindung der Formel IIa zunächst zum entsprechenden Hydroxyanilin der Formel Vb reduziert, Vb mit einer Carbonylverbindung der Formel VI in das entsprechende Anilid der Formel VII überführt, VII anschließend mit einer Verbindung VIII in das Amid der Formel IX umwandelt, IX anschließend in das entsprechende Benzylhalogenid der Formel X überführt und X in Gegenwart einer Base mit einem 3-Hydroxytriazol der Formel III in I umwandelt.

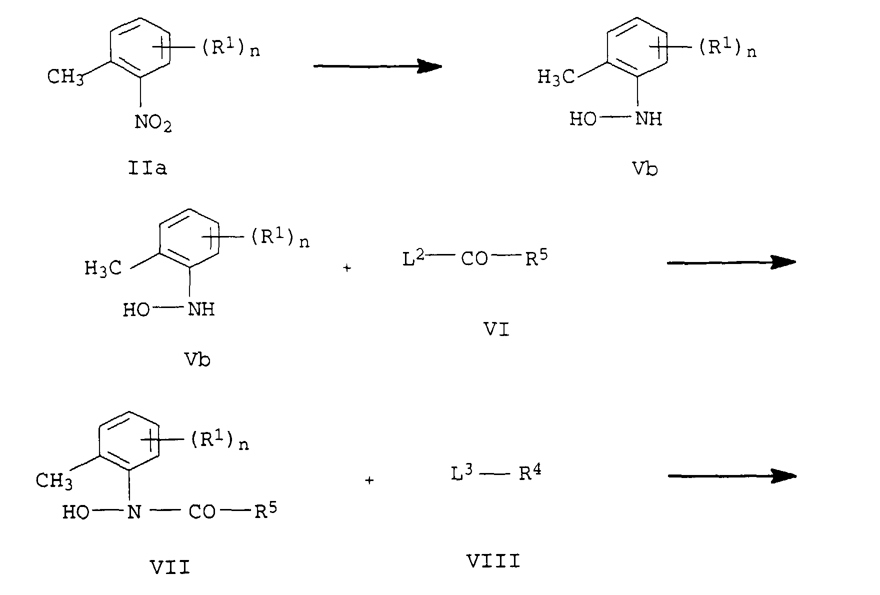

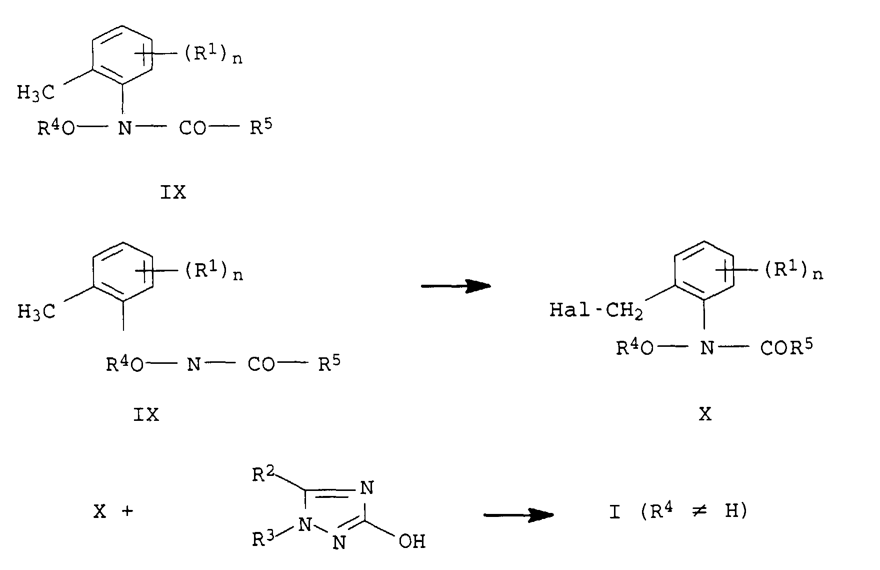

In der Formel X bedeutet Hal ein Halogenatom, insbesondere Chlor oder Brom.

L³ in der Formel VIII bedeutet eine nucleophil austauschbare Gruppe, beispielsweise Halogen (z.B. Chlor, Brom und Iod), oder ein Alkyl- oder Arylsulfonat (z.B. Methylsulfonat, Trifluormethylsulfonat, Phenylsulfonat und 4-Methylphenylsulfonat) und R⁴ steht nicht für Wasserstoff.

Die Umsetzungen erfolgen analog den vorstehend ausgeführten Verfahren.

Die Halogenierung der Verbindungen IX erfolgt radikalisch, wobei als Halogenierungsmittel beispielsweise N-Chlor- oder N-Bromsuccinimid, elementare Halogene (z.B. Chlor oder Brom) oder Thionylchlorid, Sulfurylchlorid, Phosphortri- oder Phosphorpentachlorid eingesetzt werden können. Üblicherweise verwendet man zusätzlich einen Radikalstarter (z.B. Azobisisobutyronitril) oder man führt die Umsetzung unter Bestrahlung (mit UV-Licht) durch. Die Halogenierung erfolgt in an sich bekannter Weise in einem üblichen organischen Verdünnungsmittel.

Die Verbindungen I, in denen R⁴ nicht Wasserstoff bedeutet, erhält man außerdem dadurch, daß man eine entsprechende Verbindung der Formel I, in der R⁴ Wasserstoff bedeutet, mit einer Verbindung der Formel VIII umsetzt.

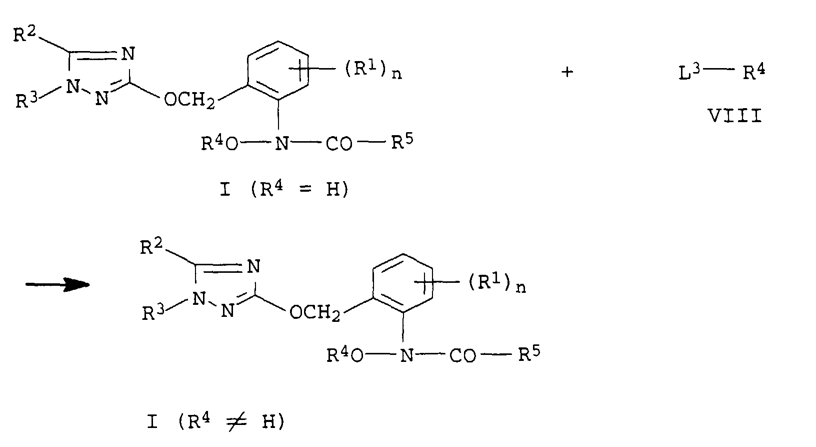

Die Umsetzung erfolgt in an sich bekannter Weise in einem inerten organischen Lösungsmittel in Gegenwart einer Base bei Temperaturen von -20°C bis 50°C.

Als Basen dienen insbesondere Natriumhydrogencarbonat, Kaliumcarbonat, Natriumhydroxid und wäßrige Natriumhydroxid Lösungen.

Als Lösungsmittel finden insbesondere Aceton, Dimethylformamid, Toluol, tert.-Butylmethylether, Essigsäureethylester und Methanol Verwendung.

Die Verbindungen der Formel I, in denen R⁵ für Methylamino steht, erhält man vorteilhaft dadurch, daß man ein Benzylanilid der Formel IXa in das entsprechende Benzylhalogenid der Formel Xa überführt, Xa in Gegenwart einer Base mit einem 3-Hydroxytriazol der Formel III in eine Verbindung der Formel I.A überführt und I.A anschließend mit Methylamin zu I umsetzt.

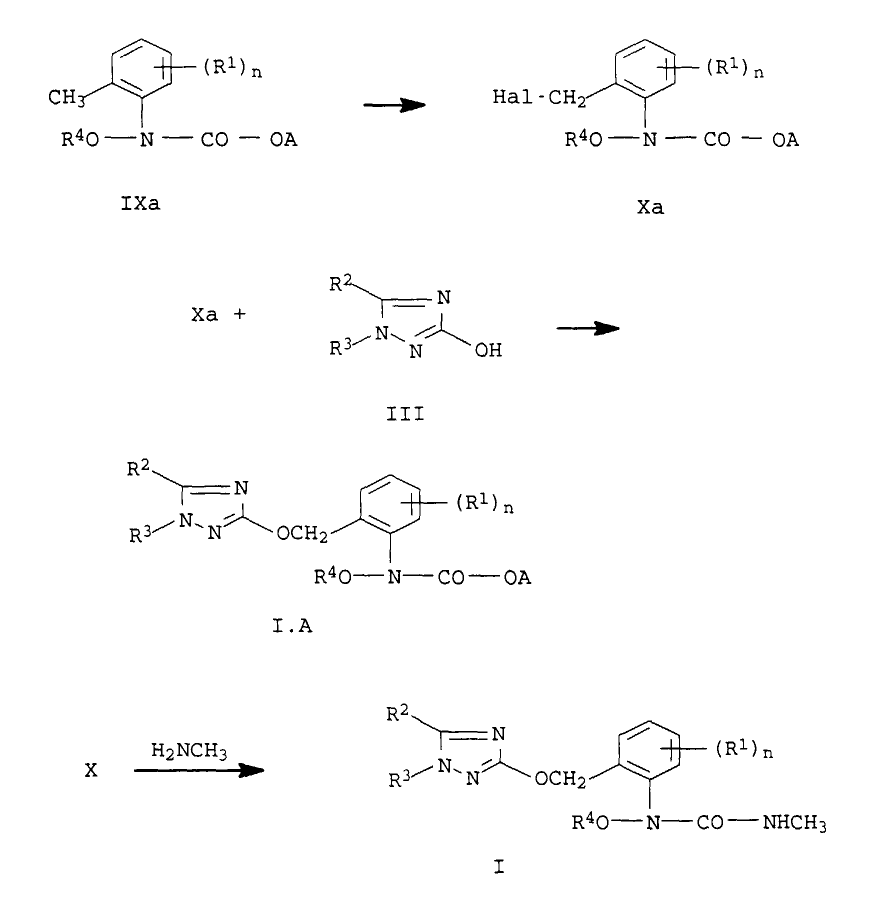

A in den Formeln IXa, Xa und I.A steht für Alkyl (insbesondere C₁-C₆-Alkyl) oder Phenyl; Hal in der Formel VIIIa steht für Halogen (insbesondere Chlor und Brom).

Die Umsetzungen von IXa nach Xa und von Xa nach I.A erfolgen im allgemeinen und im besonderen unter den vorstehend beschriebenen Bedingungen.

Die Umsetzung der Verbindungen I.A mit Methylamin erfolgt bei Temperaturen von 0°C bis 100°C in Substanz (lösungsmittelfrei) oder in einem inerten Lösungsmittel oder in einem Lösungsmittelgemisch.

Als Lösungsmittel eignen sich insbesondere Wasser, tert.-Butylmethylether und Toluol oder deren Gemische. Es kann vorteilhaft sein, zur Verbesserung der Löslichkeit der Edukte zusätzlich eines der folgenden Lösungsmittel (als Lösungsvermittler) zuzusetzen: Tetrahydrofuran, Methanol, Dimethylformamid und Ethylenglycolether.

Methylamin wird üblicherweise in einem Überschuß bis zu 100% bezogen auf die Verbindungen eingesetzt oder als Lösungsmittel verwendet. Es kann im Hinblick auf die Ausbeute vorteilhaft sein, die Umsetzung unter Druck durchzuführen.

Die Herstellung der Verbindungen I erfolgt über Zwischenprodukte der Formel XII 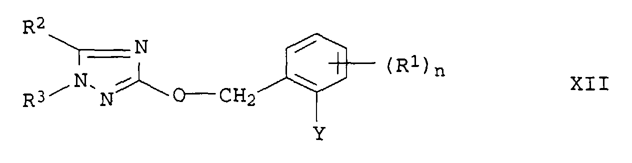 in der n und R¹, R² und R³ die in Anspruch 1 gegebene Bedeutung haben und Y für die folgenden Gruppen steht:
- Y: NO₂, NHOH oder NHOR⁴, wobei R⁴ die in Anspruch 1 gegebene Bedeutung hat.

Insbesondere sind bei der Herstellung Zwischenprodukte der Formel XII bevorzugt, in denen Y für NHOH steht.

Außerdem sind bei der Herstellung Zwischenprodukte der Formel XII bevorzugt, in denen Y für NO₂ steht.

Im Hinblick auf die Herstellung der Verbindungen I, in denen R⁵ für Methylamino steht werden Zwischenprodukte der allgemeinen Formel XIII 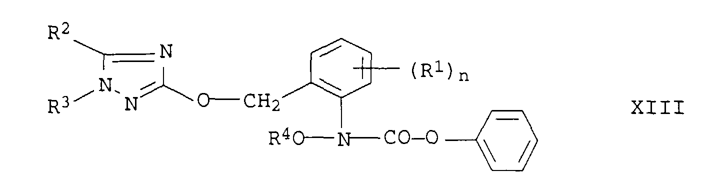 in der die Substituenten R¹, R², R³ und R⁴ sowie der Index n die in Anspruch 1 gegebene Bedeutung haben.

Gleichermaßen bevorzugt sind solche Verbindungen XIII, in denen R⁴ für Wasserstoff, Methyl oder Ethyl steht.

Daneben werden Verbindungen XIII bevorzugt, in denen n für 0 oder 1 steht.

Besonders bevorzugt sind solche Verbindungen XIII, in denen die Substituenten und der Index die folgende Bedeutung haben:
- n: 0,
- R⁴: Wasserstoff, Methyl oder Ethyl.

Die Verbindungen I können saure oder basische Zentren enthalten und dementsprechend Säureadditionsprodukte oder Basenadditionsprodukte oder Salze bilden.

Säuren für Säureadditionsprodukte sind u.a. Mineralsäuren (z.B. Halogenwasserstoffsäuren wie Chlorwasserstoff- und Bromwasserstoffsäure, Phosphorsäure, Schwefelsäure, Salpetersäure), organische Säuren (z.B. Ameisensäure, Essigsäure, Oxalsäure, Malonsäure, Milchsäure, Äpfelsäure, Bernsteinsäure, Weinsäure, Zitronensäure, Salizylsäure, p-Toluolsulfonsäure, Dodecylbenzolsulfonsäure) oder andere protonenacide Verbindungen (z.B. Saccharin).

Basen für Basenadditionsprodukte sind u.a. Oxide, Hydroxide, Carbonate oder Hydrogencarbonate von Alkalimetallen oder Erdalkalimetallen (z.B. Kalium- oder Natriumhydroxyd oder -carbonat) oder Ammoniumverbindungen (z.B. Ammoniumhydroxyd).

Bei den in den vorstehenden Formeln angegebenen Definitionen der Symbole wurden z.T. Sammelbegriffe verwendet, die allgemein repräsentativ für die folgenden Substituenten stehen:
Halogen: Fluor, Chlor, Brom und Jod;
C₁₋₄ bzw C₁₋₆-Alkyl: gesättigte, geradkettige oder verzweigte Kohlenwasserstoffreste mit 1 bis 4 oder 6 Kohlenstoffatomen, z.B. Methyl, Ethyl, Propyl, 1-Methylethyl, Butyl, 1-Methyl-propyl, 2-Methylpropyl und 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl;
C₁₋₄-Halogenalkyl: geradkettige oder verzweigte Alkylgruppen mit 1 bis 4 Kohlenstoffatomen (wie vorstehend genannt), wobei diese in Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
C₁₋₄-Alkylcarbonyl: geradkettige oder verzweigte Alkylgruppen, mit 1 bis 4 Kohlenstoffatomen (wie vorstehend genannt), welche über eine Carbonylgruppe (-CO-) an das Gerüst gebunden sind;
C₁₋₄-Alkoxy: geradkettige oder verzweigte Alkylgruppen mit 1 bis 4 Kohlenstoffatomen (wie vorstehend genannt), welche über ein Sauerstoffatom (-O-) an das Gerüst gebunden sind;
C₁₋₄-Alkoxycarbonyl: geradkettige oder verzweigte Alkoxygruppen mit 1 bis 4 Kohlenstoffatomen (wie vorstehend genannt), welche über eine Carbonylgruppe (-CO-) an das Gerüst gebunden sind;
C₁₋₄-Alkylthio: geradkettige oder verzweigte Alkylgruppen mit 1 bis 4 Kohlenstoffatomen (wie vorstehend genannt), welche über ein Schwefelatom (-S-) an das Gerüst gebunden sind;
C₃₋₆-Cycloalkyl: monocyclische Kohlenwasserstoffreste mit 3 bis 6 Kohlenstoffatomen, z.B. Cyclopropyl, Cyclobutyl, Cyclopentyl, Cyclohexyl;
Pyridyl, Pyrimidyl z.B.: 2-Pyridinyl, 3-Pyridinyl, 4-Pyridinyl, 2-Pyrimidinyl, 4-Pyrimidinyl;
Die bei den Resten genannten aromatischen oder heteroaromatischen Systeme können ihrerseits partiell oder vollständig halogeniert sein, d.h. die Wasserstoffatome dieser Gruppen können partiell oder vollständig durch Halogenatome wie Fluor, Chlor, Brom und Jod, vorzugsweise Fluor und Chlor ersetzt sein.

Diese aromatischen oder heteroaromatischen Systeme können neben den bezeichneten Halogenatomen ein bis drei der folgenden Substituenten tragen:
Nitro;
Cyano;
C₁-C₆-Alkyl wie vorstehend genannt, vorzugsweise Methyl, Ethyl, 1-Methylethyl, 1,1-Dimethylethyl, Butyl, Hexyl, insbesondere Methyl und 1-Methylethyl;
C₁-C₄-Halogenalkyl, wie vorstehend genannt, vorzugsweise Trichlormethyl, Difluormethyl, Trifluormethyl, 2,2-Difluorethyl, 2,2,2-Trifluorethyl und Pentafluorethyl;
C₁-C₄-Alkoxy, vorzugsweise Methoxy, Ethoxy, 1-Methylethoxy und 1,1-Dimethylethoxy, insbesondere Methoxy;
C₁-C₄-Halogenalkoxy, besonders C₁-C₂-Halogenalkoxy, vorzugsweise Difluormethyloxy, Trifluormethyloxy und 2,2,2-Trifluorethyloxy, insbesondere Difluormethyloxy;
C₁-C₄-Alkylcarbonyl wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl;
C₁-C₄-Alkoxycarbonyl wie Methoxycarbonyl, Ethoxycarbonyl, Propyloxycarbonyl, 1-Methyl-ethoxycarbonyl, Butyloxycarbonyl, 1-Methylpropyloxycarbonyl, 2-Methylpropyloxycarbonyl, 1,1-Dimethylethoxycarbonyl;
C₃-C₆-Cycloalkyl wie Cyclopropyl, Cyclobutyl, Cyclopentyl, Cyclohexyl und Cycloheptyl, vorzugsweise Cyclopropyl, Cyclopentyl und Cyclohexyl, insbesondere Cyclopropyl;

Im Hinblick auf ihre biologische Wirkung sind Verbindungen I bevorzugt, in denen n für 0 oder 1, insbesondere für 0, steht.

Die Erfindung betrifft Verbindungen I bevorzugt, in denen R¹ für Halogen, C₁-C₄-Alkyl, C₁-C₂-Halogenalkyl, C₁-C₄-Alkoxy oder C₁-C₂-Halogenalkoxy steht.

Ferner werden Verbindungen I bevorzugt, in denen R² Nitro, Halogen, C₁-C₄-Alkyl, C₁-C₄-Halogenalkyl, C₁-C₄-Alkoxy oder C₁-C₄-Alkoxycarbonyl steht.

Des weiteren werden Verbindungen I bevorzugt, in denen R³ für C₁-C₄-Alkyl oder C₃-C₆-Cycloalkyl steht.

Insbesondere werden Verbindungen I bevorzugt, in denen R³ für Phenyl oder Benzyl steht, wobei der Phenylrest partiell oder vollständig halogeniert sein kann und/oder
- ein bis drei der folgenden Reste: Cyano, Nitro, C₁-C₆-Alkyl, C₁-C₄-Halogenalkyl, C₁-C₄-Alkoxy, C₁-C₄-Halogenalkoxy, C₁-C₄-Alkoxy-C₁-C₄-alkyl, C₃-C₆-Cycloalkyl, C₁-C₄-Alkylcarbonyl, C₁-C₄-Alkoxycarbonyl, Phenyl, Phenoxy und Phenyl-C₁-C₄-alkoxy, wobei die Phenylringe ihrerseits partiell oder vollständig halogeniert sein können und/oder ein bis drei der folgenden Reste tragen können: Cyano, Nitro, C₁-C₄-Alkyl, C₁-C₂-Halogenalkyl, C₁-C₄-Alkoxy, C₁-C₂-Halogenalkoxy, C₃-C₆-Cycloalkyl, C₁-C₄-Alkylcarbonyl oder C₁-C₄-Alkoxycarbonyl, und/oder
- eine Gruppe CR'=NOR", in der R' Wasserstoff oder C₁-C₄-Alkyl bedeutet und R" für C₁-C₆-Alkyl steht, und/oder
- zwei benachbarte C-Atome des Phenylrings über eine Oxy-C₁-C₃-alkoxy-Brücke oder eine Oxy-C₁-C₃-halogenalkoxyBrücke
tragen kann.

Außerdem werden Verbindungen I insbesondere bevorzugt, in denen R³ für Pyridyl oder Pyrimidyl steht, wobei der heteroaromatische Ring partiell oder vollständig halogeniert sein kann und/oder ein bis drei der folgenden Reste tragen kann: Cyano, Nitro, C₁-C₄-Alkyl, C₁-C₂-Halogenalkyl, C₁-C₄-Alkoxy, C₁-C₂-Halogenalkoxy, C₃-C₆-Cycloalkyl, C₁-C₄-Alkylcarbonyl oder C₁-C₄-Alkoxycarbonyl.

Die Erfindung betrifft Verbindungen I, in denen R⁴ für Wasserstoff, C₁-C₄-Alkyl oder C₁-C₂-Halogenalkyl steht.

Beispiele für insbesondere bevorzugte Verbindungen I sind in den Tabellen zusammengestellt.

### Tabelle 1

Verbindungen der allgemeinen Formel I.1, in denen R⁴ für Methyl steht, R⁵ Methyl bedeutet und R^{x} ₚ für eine Verbindung einer Zeile der Tabelle A entspricht

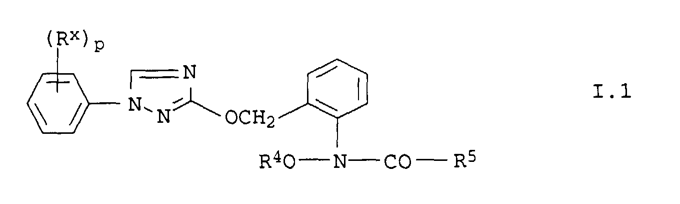

### Tabelle 2

Verbindungen der allgemeinen Formel I.1, in denen R⁴ für Methyl steht, R⁵ Ethyl bedeutet und RXp für eine Verbindung einer Zeile der Tabelle A entspricht

### Tabelle 3

Verbindungen der allgemeinen Formel I.1, in denen R⁴ für Methyl steht, R⁵ Methoxy bedeutet und R^{x}ₚ für eine Verbindung einer Zeile der Tabelle A entspricht

### Tabelle 4

Verbindungen der allgemeinen Formel I.1, in denen R⁴ für Methyl steht, R⁵ Methylamino bedeutet und R^{x}ₚ für eine Verbindung einer Zeile der Tabelle A entspricht

### Tabelle 5

Verbindungen der allgemeinen Formel I.2, in denen R⁴ für Methyl steht, R⁵ Methyl bedeutet, R² Methyl bedeutet und R^{x}ₚ für eine Verbindung einer Zeile der Tabelle A entspricht

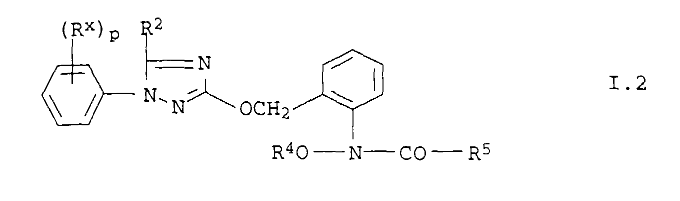

### Tabelle 6

Verbindungen der allgemeinen Formel I.2, in denen R⁴ für Methyl steht, R⁵ Ethyl bedeutet, R² Methyl bedeutet und R^{x}ₚ für eine Verbindung einer Zeile der Tabelle A entspricht

### Tabelle 7

Verbindungen der allgemeinen Formel I.2, in denen R⁴ für Methyl steht, R⁵ Methoxy bedeutet, R² Methyl bedeutet und R^{x}ₚ für eine Verbindung einer Zeile der Tabelle A entspricht

### Tabelle 8

Verbindungen der allgemeinen Formel I.2, in denen R⁴ für Methyl steht, R⁵ Methylamino bedeutet, R² Methyl bedeutet und R^{x}ₚ für eine Verbindung einer Zeile der Tabelle A entspricht

### Tabelle 9

Verbindungen der allgemeinen Formel I.2, in denen R⁴ für Methyl steht, R⁵ Methyl bedeutet, R² Ethyl bedeutet und R^{x}ₚ für eine Verbindung einer Zeile der Tabelle A entspricht

### Tabelle 10

Verbindungen der allgemeinen Formel I.2, in denen R⁴ für Methyl steht, R⁵ Ethyl bedeutet, R² Ethyl bedeutet und R^{x}ₚ für eine Verbindung einer Zeile der Tabelle A entspricht

### Tabelle 11

Verbindungen der allgemeinen Formel I.2, in denen R⁴ für Methyl steht, R⁵ Methoxy bedeutet, R² Ethyl bedeutet und R^{x}ₚ für eine Verbindung einer Zeile der Tabelle A entspricht

### Tabelle 12

Verbindungen der allgemeinen Formel I.2, in denen R⁴ für Methyl steht, R⁵ Methylamino bedeutet, R² Ethyl bedeutet und R^{x}ₚ für eine Verbindung einer Zeile der Tabelle A entspricht

### Tabelle 13

Verbindungen der allgemeinen Formel I.2, in denen R⁴ für Methyl steht, R⁵ Methyl bedeutet, R² Chlor bedeutet und R^{x}ₚ für eine Verbindung einer Zeile der Tabelle A entspricht

### Tabelle 14

Verbindungen der allgemeinen Formel I.2, in denen R⁴ für Methyl steht, R⁵ Ethyl bedeutet, R² Chlor bedeutet und R^{x}ₚ für eine Verbindung einer Zeile der Tabelle A entspricht

### Tabelle 15

Verbindungen der allgemeinen Formel I.2, in denen R⁴ für Methyl steht, R⁵ Methoxy bedeutet, R² Chlor bedeutet und R^{x}ₚ für eine Verbindung einer Zeile der Tabelle A entspricht

### Tabelle 16

Verbindungen der allgemeinen Formel I.2, in denen R⁴ für Methyl steht, R⁵ Methylamino bedeutet, R² Chlor bedeutet und R^{x}ₚ für eine Verbindung einer Zeile der Tabelle A entspricht

### Tabelle 17

Verbindungen der allgemeinen Formel I.2, in denen R⁴ für Methyl steht, R⁵ Methyl bedeutet, R² Brom bedeutet und R^{x}ₚ für eine Verbindung einer Zeile der Tabelle A entspricht

### Tabelle 18

Verbindungen der allgemeinen Formel I.2, in denen R⁴ für Methyl steht, R⁵ Ethyl bedeutet, R² Brom bedeutet und R^{x}ₚ für eine Verbindung einer Zeile der Tabelle A entspricht

### Tabelle 19

Verbindungen der allgemeinen Formel I.2, in denen R⁴ für Methyl steht, R⁵ Methoxy bedeutet, R² Brom bedeutet und R^{x}ₚ für eine Verbindung einer Zeile der Tabelle A entspricht

### Tabelle 20

Verbindungen der allgemeinen Formel I.2, in denen R⁴ für Methyl steht, R⁵ Methylamino bedeutet, R² Brom bedeutet und R^{x}ₚ für eine Verbindung einer Zeile der Tabelle A entspricht

### Tabelle 21

Verbindungen der allgemeinen Formel I.1, in denen R⁴ für Wasserstoff steht, R⁵ Methyl bedeutet und R^{x}ₚ für eine Verbindung einer Zeile der Tabelle A entspricht

### Tabelle 22

Verbindungen der allgemeinen Formel I.1, in denen R⁴ für Wasserstoff steht, R⁵ Ethyl bedeutet und R^{x}ₚ für eine Verbindung einer Zeile der Tabelle A entspricht

### Tabelle 23

Verbindungen der allgemeinen Formel I.1, in denen R⁴ für Wasserstoff steht, R⁵ Methoxy bedeutet und R^{x}ₚ für eine Verbindung einer Zeile der Tabelle A entspricht

### Tabelle 24

Verbindungen der allgemeinen Formel I.1, in denen R⁴ für Wasserstoff steht, R⁵ Methylamino bedeutet und R^{x}ₚ für eine Verbindung einer Zeile der Tabelle A entspricht

### Tabelle 25

Verbindungen der allgemeinen Formel I.2, in denen R⁴ für Wasserstoff steht, R⁵ Methyl bedeutet, R² Methyl bedeutet und R^{x}ₚ für eine Verbindung einer Zeile der Tabelle A entspricht

### Tabelle 26

Verbindungen der allgemeinen Formel I.2, in denen R⁴ für Wasserstoff steht, R⁵ Ethyl bedeutet, R² Methyl bedeutet und R^{x}ₚ für eine Verbindung einer Zeile der Tabelle A entspricht

### Tabelle 27

Verbindungen der allgemeinen Formel I.2, in denen R⁴ für Wasserstoff steht, R⁵ Methoxy bedeutet, R² Methyl bedeutet und R^{x}ₚ für eine Verbindung einer Zeile der Tabelle A entspricht Tabelle 28

Verbindungen der allgemeinen Formel I.2, in denen R⁴ für Wasserstoff steht, R⁵ Methylamino bedeutet, R² Methyl bedeutet und R^{x}ₚ für eine Verbindung einer Zeile der Tabelle A entspricht

### Tabelle 29

Verbindungen der allgemeinen Formel I.2, in denen R⁴ für Wasserstoff steht, R⁵ Methyl bedeutet, R² Ethyl bedeutet und R^{x}ₚ für eine Verbindung einer Zeile der Tabelle A entspricht

### Tabelle 30

Verbindungen der allgemeinen Formel I.2, in denen R⁴ für Wasserstoff steht, R⁵ Ethyl bedeutet, R² Ethyl bedeutet und R^{x}ₚ für eine Verbindung einer Zeile der Tabelle A entspricht

### Tabelle 31

Verbindungen der allgemeinen Formel I.2, in denen R⁴ für Wasserstoff steht, R⁵ Methoxy bedeutet, R² Ethyl bedeutet und R^{x}ₚ für eine Verbindung einer Zeile der Tabelle A entspricht

### Tabelle 32

Verbindungen der allgemeinen Formel I.2, in denen R⁴ für Wasserstoff steht, R⁵ Methylamino bedeutet, R² Ethyl bedeutet und R^{x}ₚ für eine Verbindung einer Zeile der Tabelle A entspricht

### Tabelle 33

Verbindungen der allgemeinen Formel I.2, in denen R⁴ für Wasserstoff steht, R⁵ Methyl bedeutet, R² Chlor bedeutet und R^{x}ₚ für eine Verbindung einer Zeile der Tabelle A entspricht

### Tabelle 34

Verbindungen der allgemeinen Formel I.2, in denen R⁴ für Wasserstoff steht, R⁵ Ethyl bedeutet, R² Chlor bedeutet und R^{x}ₚ für eine Verbindung einer Zeile der Tabelle A entspricht

### Tabelle 35

Verbindungen der allgemeinen Formel I.2, in denen R⁴ für Wasserstoff steht, R⁵ Methoxy bedeutet, R² Chlor bedeutet und R^{x}ₚ für eine Verbindung einer Zeile der Tabelle A entspricht

### Tabelle 36

Verbindungen der allgemeinen Formel I.2, in denen R⁴ für Wasserstoff steht, R⁵ Methylamino bedeutet, R² Chlor bedeutet und R^{x}ₚ für eine Verbindung einer Zeile der Tabelle A entspricht

### Tabelle 37

Verbindungen der allgemeinen Formel I.2, in denen R⁴ für Wasserstoff steht, R⁵ Methyl bedeutet, R² Brom bedeutet und R^{x}ₚ für eine Verbindung einer Zeile der Tabelle A entspricht

### Tabelle 38

Verbindungen der allgemeinen Formel I.2, in denen R⁴ für Wasserstoff steht, R⁵ Ethyl bedeutet, R² Brom bedeutet und R^{x}ₚ für eine Verbindung einer Zeile der Tabelle A entspricht

### Tabelle 39

Verbindungen der allgemeinen Formel I.2, in denen R⁴ für Wasserstoff steht, R⁵ Methoxy bedeutet, R² Brom bedeutet und R^{x}ₚ für eine Verbindung einer Zeile der Tabelle A entspricht

### Tabelle 40

Verbindungen der allgemeinen Formel I.2, in denen R⁴ für Wasserstoff steht, R⁵ Methylamino bedeutet, R² Brom bedeutet und R^{x}ₚ für eine Verbindung einer Zeile der Tabelle A entspricht

### Tabelle 41

Verbindungen der allgemeinen Formel I.3, in denen R⁵ Methyl bedeutet und die Kombination der Substituenten R¹, R², R³ und R⁴ für eine Verbindung jeweils einer Zeile der Tabelle B entspricht

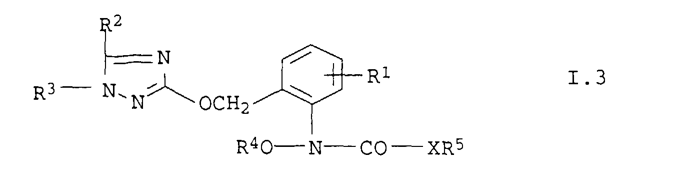

### Tabelle 42

Verbindungen der allgemeinen Formel I.3, in denen R⁵ Ethyl bedeu tet und die Kombination der Substituenten R¹, R², R³ und R⁴ für eine Verbindung jeweils einer Zeile der Tabelle B entspricht

### Tabelle 43

Verbindungen der allgemeinen Formel I.3, in denen R⁵ Methoxy bedeutet und die Kombination der Substituenten R¹, R², R³ und R⁴ für eine Verbindung jeweils einer Zeile der Tabelle B entspricht

### Tabelle 44

Verbindungen der allgemeinen Formel I.3, in denen R⁵ Methylamino und bedeutet und die Kombination der Substituenten R¹, R², R³ und R⁴ für eine Verbindung jeweils einer Zeile der Tabelle B entspricht

### Tabelle 45

Verbindungen der allgemeinen Formel I.2, in denen R⁴ für Methyl steht, R⁵ Methyl bedeutet, R² Cyano bedeutet und R^{x}ₚ für eine Verbindung einer Zeile der Tabelle A entspricht

### Tabelle 46

Verbindungen der allgemeinen Formel I.2, in denen R⁴ für Methyl steht, R⁵ Ethyl bedeutet, R² Cyano bedeutet und R^{x}ₚ für eine Verbindung einer Zeile der Tabelle A entspricht

### Tabelle 47

Verbindungen der allgemeinen Formel I.2, in denen R⁴ für Methyl steht, R⁵ Methoxy bedeutet, R² Cyano bedeutet und R^{x}ₚ für eine Verbindung einer Zeile der Tabelle A entspricht

### Tabelle 48

Verbindungen der allgemeinen Formel I.2, in denen R⁴ für Methyl steht, R⁵ Methylamino bedeutet, R² Cyano bedeutet und R^{x}ₚ für eine Verbindung einer Zeile der Tabelle A entspricht

### Tabelle 49

Verbindungen der allgemeinen Formel I.2, in denen R⁴ für Methyl steht, R⁵ Methyl bedeutet, R² Methoxy bedeutet und R^{x}ₚ für eine Verbindung einer Zeile der Tabelle A entspricht

### Tabelle 50

Verbindungen der allgemeinen Formel I.2, in denen R⁴ für Methyl steht, R⁵ Ethyl bedeutet, R² Methoxy bedeutet und R^{x}ₚ für eine Verbindung einer Zeile der Tabelle A entspricht

### Tabelle 51

Verbindungen der allgemeinen Formel I.2, in denen R⁴ für Methyl steht, R⁵ Methoxy bedeutet, R² Methoxy bedeutet und R^{x}ₚ für eine Verbindung einer Zeile der Tabelle A entspricht

### Tabelle 52

Verbindungen der allgemeinen Formel I.2, in denen R⁴ für Methyl steht, R⁵ Methylamino bedeutet, R² Methoxy bedeutet und R^{x}ₚ für eine Verbindung einer Zeile der Tabelle A entspricht

### Tabelle 53

Verbindungen der allgemeinen Formel I.2, in denen R⁴ für Methyl steht, R⁵ Methyl bedeutet, R² Ethoxy bedeutet und R^{x}ₚ für eine Verbindung einer Zeile der Tabelle A entspricht

### Tabelle 54

Verbindungen der allgemeinen Formel I.2, in denen R⁴ für Methyl steht, R⁵ Ethyl bedeutet, R² Ethoxy bedeutet und R^{x}ₚ für eine Verbindung einer Zeile der Tabelle A entspricht

### Tabelle 55

Verbindungen der allgemeinen Formel I.2, in denen R⁴ für Methyl steht, R⁵ Methoxy bedeutet, R² Ethoxy bedeutet und R^{x}ₚ für eine Verbindung einer Zeile der Tabelle A entspricht

### Tabelle 56

Verbindungen der allgemeinen Formel I.2, in denen R⁴ für Methyl steht, R⁵ Methylamino bedeutet, R² Ethoxy bedeutet und R^{x}ₚ für eine Verbindung einer Zeile der Tabelle A entspricht

### Tabelle 57

Verbindungen der allgemeinen Formel I.2, in denen R⁴ für Methyl steht, R⁵ Methyl bedeutet, R² n-Propoxy bedeutet und R^{x}ₚ für eine Verbindung einer Zeile der Tabelle A entspricht

### Tabelle 58

Verbindungen der allgemeinen Formel I.2, in denen R⁴ für Methyl steht, R⁵ Ethyl bedeutet, R² n-Propoxy bedeutet und R^{x}ₚ für eine Verbindung einer Zeile der Tabelle A entspricht

### Tabelle 59

Verbindungen der allgemeinen Formel I.2, in denen R⁴ für Methyl steht, R⁵ Methoxy bedeutet, R² n-Propoxy bedeutet und R^{x}ₚ für eine Verbindung einer Zeile der Tabelle A entspricht

### Tabelle 60

Verbindungen der allgemeinen Formel I.2, in denen R⁴ für Methyl steht, R⁵ Methylamino bedeutet, R² n-Propoxy bedeutet und R^{x}ₚ für eine Verbindung einer Zeile der Tabelle A entspricht

### Tabelle 61

Verbindungen der allgemeinen Formel I.2, in denen R⁴ für Methyl steht, R⁵ Methyl bedeutet, R² CF₃ bedeutet und R^{x}ₚ für eine Verbindung einer Zeile der Tabelle A entspricht

### Tabelle 62

Verbindungen der allgemeinen Formel I.2, in denen R⁴ für Methyl steht, R⁵ Ethyl bedeutet, R² CF₃ bedeutet und R^{x}ₚ für eine Verbindung einer Zeile der Tabelle A entspricht

### Tabelle 63

Verbindungen der allgemeinen Formel I.2, in denen R⁴ für Methyl steht, R⁵ Methoxy bedeutet, R² CF₃ bedeutet und R^{x}ₚ für eine Verbindung einer Zeile der Tabelle A entspricht

### Tabelle 64

Verbindungen der allgemeinen Formel I.2, in denen R⁴ für Methyl steht, R⁵ Methylamino bedeutet, R² CF₃ bedeutet und R^{x}ₚ für eine Verbindung einer Zeile der Tabelle A entspricht

**Tabelle A**

| Nr. | R^{x}_{P} |
|---|---|
| 1 | H |
| 2 | 2-F |
| 3 | 3-F |
| 4 | 4-F |
| 5 | 2,4-F₂ |
| 6 | 2,4,6-F₃ |
| 7 | 2,3,4,5,6-F₅ |
| 8 | 2,3-F₂ |
| 9 | 2-Cl |
| 10 | 3-Cl |
| 11 | 4-Cl |
| 12 | 2,3-Cl₂ |
| 13 | 2,4-Cl₂ |
| 14 | 2,5-Cl₂ |
| 15 | 2,6-Cl₂ |
| 16 | 3,4-Cl₂ |
| 17 | 3,5-Cl₂ |
| 18 | 2,3,4-Cl₃ |
| 19 | 2,3,5-Cl₃ |
| 20 | 2,3,6-Cl₃ |
| 21 | 2,4,5-Cl₃ |
| 22 | 2,4,6-Cl₃ |
| 23 | 3,4,5-Cl₃ |
| 24 | 2,3,4,6-Cl₄ |
| 25 | 2,3,5,6-Cl₄ |
| 26 | 2,3,4,5,6-Cl₅ |
| 27 | 2-Br |
| 28 | 3-Br |
| 29 | 4-Br |
| 30 | 2,4-Br₂ |
| 31 | 2,5-Br₂ |
| 32 | 2,6-Br₂ |
| 33 | 2,4,6-Br₃ |
| 34 | 2,3,4,5,6-Br₅ |
| 35 | 2-J |
| 36 | 3-J |
| 37 | 4-J |
| 38 | 2,4-J₂ |
| 39 | 2-Cl, 3-F |
| 40 | 2-Cl, 4-F |
| 41 | 2-Cl, 5-F |
| 42 | 2-Cl, 6-F |
| 43 | 2-Cl, 3-Br |
| 44 | 2-Cl, 4-Br |
| 45 | 2-Cl, 5-Br |
| 46 | 2-Cl, 6-Br |
| 47 | 2-Br, 3-Cl |
| 48 | 2-Br, 4-Cl |
| 49 | 2-Br, 5-Cl |
| 50 | 2-Br, 3-F |
| 51 | 2-Br, 4-F |
| 52 | 2-Br, 5-F |
| 53 | 2-Br, 6-F |
| 54 | 2-F, 3-Cl |
| 55 | 2-F, 4-Cl |
| 56 | 2-F, 5-Cl |
| 57 | 3-Cl, 4-F |
| 58 | 3-Cl, 5-F |
| 59 | 3-Cl, 4-Br |
| 60 | 3-Cl, 5-Br |
| 61 | 3-F, 4-Cl |
| 62 | 3-F, 4-Br |
| 63 | 3-Br, 4-Cl |
| 64 | 3-Br, 4-F |
| 65 | 2,6-Cl₂, 4-Br |
| 66 | 2-CH₃ |
| 67 | 3-CH₃ |
| 68 | 4-CH₃ |
| 69 | 2,3-(CH₃)₂ |
| 70 | 2,4-(CH₃)₂ |
| 71 | 2,5-(CH₃)₂ |
| 72 | 2,6-(CH₃)₂ |
| 73 | 3,4-(CH₃)₂ |
| 74 | 3,5-(CH₃)₂ |
| 75 | 2,3,5-(CH₃)₃ |
| 76 | 2,3,4-(CH₃)₃ |
| 77 | 2,3,6-(CH₃)₃ |
| 78 | 2,4,5-(CH₃)₃ |
| 79 | 2,4,6-(CH₃)₃ |
| 80 | 3,4,5-(CH₃)₃ |
| 81 | 2-C₂H₅ |
| 82 | 3-C₂H₅ |
| 83 | 4-C₂H₅ |
| 84 | 2,4-(C₂H₅)₂ |
| 85 | 2,6-(C₂H₅)₂ |
| 86 | 3,5-(C₂H₅)₂ |
| 87 | 2,4,6-(C₂H₅)₃ |
| 88 | 2-n-C₃H₇ |
| 89 | 3-n-C₃H₇ |
| 90 | 4-n-C₃H₇ |
| 91 | 2-i-C₃H₇ |
| 92 | 3-i-C₃H₇ |
| 93 | 4-i-C₃H₇ |
| 94 | 2,4-(1-C₃H₇)₂ |
| 95 | 2,6-(1-C₃H₇)₂ |
| 96 | 3,5-(i-C₃H₇)₂ |
| 97 | 2-s-C₄H₉ |
| 98 | 3-s-C₄H₉ |
| 99 | 4-s-C₄H₉ |
| 100 | 2-t-C₄H₉ |
| 101 | 3-t-C₄H₉ |
| 102 | 4-t-C₄H₉ |
| 103 | 2-CH₃, 4-t-C₄H₉ |
| 104 | 2-CH₃, 6-t-C₄H₉ |
| 105 | 2-CH₃, 4-i-C₃H₇ |
| 106 | 2-CH₃, 5-i-C₃H₇ |
| 107 | 3-CH₃, 4-i-C₃H₇ |
| 108 | 2-cyclo-C₆H₁₁ |
| 109 | 3-cyclo-C₆H₁₁ |
| 110 | 4-cyclo-C₆H₁₁ |
| 111 | 2-Cl, 4-C₆H₅ |
| 112 | 2-Br, 4-C₆H₅ |
| 113 | 2-OCH₃ |
| 114 | 3-OCH₃ |
| 115 | 4-OCH₃ |
| 116 | 2-OC₂H₅ |
| 117 | 3-O-C₂H₅ |
| 118 | 4-O-C₂H₅ |
| 119 | 2-O-n-C₃H₇ |
| 120 | 3-O-n-C₃H₇ |
| 121 | 4-O-n-C₃H₇ |
| 122 | 2-O-i-C₃H₇ |
| 123 | 3-O-i-C₃H₇ |
| 124 | 4-O-i-C₃H₇ |
| 125 | 2-O-CH₂C₆H₅ |
| 126 | 3-O-CH₂C₆H₅ |
| 127 | 4-O-CH₂C₆H₅ |
| 128 | 2-O-(CH₂)₃C₆H₅ |
| 129 | 4-O-(CH₂)₃C₆H₅ |
| 130 | 2,3-(OCH₃)₂ |
| 131 | 2,4-(OCH₃)₂ |
| 132 | 2,5-(OCH₃)₂ |
| 133 | 2,6-(OCH₃)₂ |
| 134 | 3,4-(OCH₃)₂ |
| 135 | 3,5-(OCH₃)₂ |
| 136 | 2-O-t-C₄H₉ |
| 137 | 3-O-t-C₄H₉ |
| 138 | 4-O-t-C₄H₉ |
| 139 | 3-(3'-Cl-C₆H₄) |
| 140 | 4-(4'-CH₃-C₆H₄) |
| 141 | 2-O-C₆H₅ |
| 142 | 3-O-C₆H₅ |
| 143 | 4-O-C₆H₅ |
| 144 | 2-O-(2'-F-C₆H₄) |
| 145 | 3-O-(3'-Cl-C₆H₄) |
| 146 | 4-O-(4'-CH₃-C₆H₄) |
| 147 | 2,3,6-(CH₃)₃, 4-F |
| 148 | 2,3,6-(CH₃)₃, 4-Cl |
| 149 | 2,3,6-(CH₃)₃, 4-Br |
| 150 | 2,4-(CH₃)₂, 6-F |
| 151 | 2,4-(CH₃)₂, 6-Cl |
| 152 | 2,4-(CH₃)₂, 6-Br |
| 153 | 2-i-C₃H₇, 4-Cl, 5-CH₃ |
| 154 | 2-Cl, 4-NO₂ |
| 155 | 2-NO₂, 4-Cl |
| 156 | 2-OCH₃, 5-NO₂ |
| 157 | 2,4-Cl₂, 5-NO₂ |
| 158 | 2,4-Cl₂, 6-NO₂ |
| 159 | 2,6-Cl₂, 4-NO₂ |
| 160 | 2,6-Br₂, 4-NO₂ |
| 161 | 2,6-J₂, 4-NO₂ |
| 162 | 2-CH₃, 5-i-C₃H₇, 4-Cl |
| 163 | 2-CO₂CH₃ |
| 164 | 3-CO₂CH₃ |
| 165 | 4-CO₂CH₃ |
| 166 | 2-CH₂-OCH₃ |
| 167 | 3-CH₂-OCH₃ |
| 168 | 4-CH₂-OCH₃ |
| 169 | 2-Me-4-CH₃-CH(CH₃)-CO |
| 170 | 2-CH₃-4-(CH₃-C=NOCH₃) |
| 171 | 2-CH₃-4-(CH₃-C=NOC₂H₅) |
| 172 | 2-CH₃-4-(CH₃-C=NO-n-C₃H₇) |
| 173 | 2-CH₃-4-(CH₃-C=NO-i-C₃H₇) |
| 174 | 2,5-(CH₃)₂-4-(CH₃-C=NOCH₃) |
| 175 | 2,5-(CH₃)₂-4-(CH₃-C=NOC₂H₅) |
| 176 | 2,5-(CH₃-4-(CH₃-C=NO-n-C₃H₇) |
| 177 | 2,5-(CH₃)₂-4-(CH₃-C=NO-i-C₃H₇) |
| 178 | 2-C₆H₅ |
| 179 | 3-C₆H₅ |
| 180 | 4-C₆H₅ |
| 181 | 2-(2'-F-C₆H₄) |
| 182 | 2-CH₃, 5-Br |
| 183 | 2-CH₃, 6-Br |
| 184 | 2-Cl, 3-CH₃ |
| 185 | 2-Cl, 4-CH₃ |
| 186 | 2-Cl, 5-CH₃ |
| 187 | 2-F, 3-CH₃ |
| 188 | 2-F, 4-CH₃ |
| 189 | 2-F, 5-CH₃ |
| 190 | 2-Br, 3-CH₃ |
| 191 | 2-Br, 4-CH₃ |
| 192 | 2-Br, 5-CH₃ |
| 193 | 3-CH₃, 4-Cl |
| 194 | 3-CH₃, 5-Cl |
| 195 | 3-CH₃, 4-F |
| 196 | 3-CH₃, 5-F |
| 197 | 3-CH₃, 4-Br |
| 198 | 3-CH₃, 5-Br |
| 199 | 3-F, 4-CH₃ |
| 200 | 3-Cl, 4-CH₃ |
| 201 | 3-Br, 4-CH₃ |
| 202 | 2-Cl, 4,5-(CH₃)₂ |
| 203 | 2-Br, 4,5-(CH₃)₂ |
| 204 | 2-Cl, 3,5-(CH₃)₂ |
| 205 | 2-Br, 3,5-(CH₃)₂ |
| 206 | 2,6-Cl₂, 4-CH₃ |
| 207 | 2,6-F₂, 4-CH₃ |
| 208 | 2,6-Br₂, 4-CH₃ |
| 209 | 2,4-Br₂, 6-CH₃ |
| 210 | 2,4-F₂, 6-CH₃ |
| 211 | 2,4-Br₂, 6-CH₃ |
| 212 | 2,6-(CH₃)₂, 4-F |
| 213 | 2,6-(CH₃)₂, 4-Cl |
| 214 | 2,6-(CH₃)₂, 4-Br |
| 215 | 3,5-(CH₃)₂, 4-F |
| 216 | 3,5-(CH₃)₂, 4-Cl |
| 217 | 3,5-(CH₃)₂, 4-Br |
| 218 | 2-CF₃ |
| 219 | 3-CF₃ |
| 220 | 4-CF₃ |
| 221 | 2-OCF₃ |
| 222 | 3-OCF₃ |
| 223 | 4-OCF₃ |
| 224 | 3-OCH₂CHF₂ |
| 225 | 2-NO₂ |
| 226 | 3-NO₂ |
| 227 | 4-NO₂ |
| 228 | 2-CN |
| 229 | 3-CN |
| 230 | 4-CN |
| 231 | 2-CH₃, 3-Cl |
| 232 | 2-CH₃, 4-Cl |
| 233 | 2-CH₃, 5-Cl |
| 234 | 2-CH₃, 6-Cl |
| 235 | 2-CH₃, 3-F |
| 236 | 2-CH₃, 4-F |
| 237 | 2-CH₃, 5-F |
| 238 | 2-CH₃, 6-F |
| 239 | 2-CH₃, 3-Br |
| 240 | 2-CH₃, 4-Br |
| 241 | 2-Pyridyl-2' |
| 242 | 3-Pyridyl-3' |
| 243 | 4-Pyridyl-4' |

**Tabelle B**

| Nr. | R¹ | R² | R³ | R⁴ |
|---|---|---|---|---|
| 1 | H | H | Cyclohexyl | CH₃ |
| 2 | H | H | Benzyl | CH₃ |
| 3 | H | H | 2-Pyridyl | CH₃ |
| 4 | H | H | 5-Cl-pyridyl-2 | CH₃ |
| 5 | H | H | 5-CF₃-pyridyl-2 | CH₃ |
| 6 | H | Cl | Cyclohexyl | CH₃ |
| 7 | H | Cl | Benzyl | CH₃ |
| 8 | H | Cl | 2-Pyridyl | CH₃ |
| 9 | H | Cl | 5-Cl-pyridyl-2 | CH₃ |
| 10 | H | Cl | 5-CF₃-pyridyl-2 | CH₃ |
| 11 | H | CH₃ | Cyclohexyl | CH₃ |
| 12 | H | CH₃ | Benzyl | CH₃ |
| 13 | H | CH₃ | 2-Pyridyl | CH₃ |
| 14 | H | CH₃ | 5-Cl-pyridyl-2 | CH₃ |
| 15 | H | CH₃ | 5-CF₃-pyridyl-2 | CH₃ |
| 16 | H | H | Cyclohexyl | C₂H₅ |
| 17 | H | H | Benzyl | C₂H₅ |
| 18 | H | H | Phenyl | C₂H₅ |
| 19 | H | H | 2-Pyridyl | C₂H₅ |
| 20 | H | H | 5-Cl-pyridyl-2 | C₂H₅ |
| 21 | H | H | 5-CF₃-pyridyl-2 | C₂H₅ |
| 22 | H | Cl | Cyclohexyl | C₂H₅ |
| 23 | H | Cl | Benzyl | C₂H₅ |
| 24 | H | Cl | Phenyl | C₂H₅ |
| 25 | H | Cl | 2-Pyridyl | C₂H₅ |
| 26 | H | Cl | 5-Cl-pyridyl-2 | C₂H₅ |
| 27 | H | Cl | 5-CF₃-pyridyl-2 | C₂H₅ |
| 28 | H | CH₃ | Cyclohexyl | C₂H₅ |
| 29 | H | CH₃ | Benzyl | C₂H₅ |
| 30 | H | CH₃ | Phenyl | C₂H₅ |
| 31 | H | CH₃ | 2-Pyridyl | C₂H₅ |
| 32 | H | CH₃ | 5-Cl-pyridyl-2 | C₂H₅ |
| 33 | H | CH₃ | 5-CF₃-pyridyl-2 | C₂H₅ |
| 34 | 3-F | H | Cyclohexyl | CH₃ |
| 35 | 3-F | H | Benzyl | CH₃ |
| 36 | 3-F | H | Phenyl | CH₃ |
| 37 | 3-F | H | 2-Pyridyl | CH₃ |
| 38 | 3-F | H | 5-Cl-pyridyl-2 | CH₃ |
| 39 | 3-F | H | 5-CF₃-pyridyl-2 | CH₃ |
| 40 | 3-F | Cl | Cyclohexyl | CH₃ |
| 41 | 3-F | Cl | Benzyl | CH₃ |
| 42 | 3-F | Cl | Phenyl | CH₃ |
| 43 | 3-F | Cl | 2-Pyridyl | CH₃ |
| 44 | 3-F | Cl | 5-Cl-pyridyl-2 | CH₃ |
| 45 | 3-F | Cl | 5-CF₃-pyridyl-2 | CH₃ |
| 46 | 3-F | CH₃ | Cyclohexyl | CH₃ |
| 47 | 3-F | CH₃ | Benzyl | CH₃ |
| 48 | 3-F | CH₃ | Phenyl | CH₃ |
| 49 | 3-F | CH₃ | 2-Pyridyl | CH₃ |
| 50 | 3-F | CH₃ | 5-Cl-pyridyl-2 | CH₃ |
| 51 | 3-F | CH₃ | 5-CF₃-pyridyl-2 | CH₃ |
| 52 | 3-F | H | Cyclohexyl | C₂H₅ |
| 53 | 3-F | H | Benzyl | C₂H₅ |
| 54 | 3-F | H | Phenyl | C₂H₅ |
| 55 | 3-F | H | 2-Pyridyl | C₂H₅ |
| 56 | 3-F | H | 5-Cl-pyridyl-2 | C₂H₅ |
| 57 | 3-F | H | 5-CF₃-pyridyl-2 | C₂H₅ |
| 58 | 3-F | Cl | Cyclohexyl | C₂H₅ |
| 59 | 3-F | Cl | Benzyl | C₂H₅ |
| 60 | 3-F | Cl | Phenyl | C₂H₅ |
| 61 | 3-F | Cl | 2-Pyridyl | C₂H₅ |
| 62 | 3-F | Cl | 5-Cl-pyridyl-2 | C₂H₅ |
| 63 | 3-F | Cl | 5-CF₃-pyridyl-2 | C₂H₅ |
| 64 | 3-F | CH₃ | Cyclohexyl | C₂H₅ |
| 65 | 3-F | CH₃ | Benzyl | C₂H₅ |
| 66 | 3-F | CH₃ | Phenyl | C₂H₅ |
| 67 | 3-F | CH₃ | 2-Pyridyl | C₂H₅ |
| 68 | 3-F | CH₃ | 5-Cl-pyridyl-2 | C₂H₅ |
| 69 | 3-F | CH₃ | 5-CF₃-pyridyl-2 | C₂H₅ |
| 70 | 6-Cl | H | Cyclohexyl | CH₃ |
| 71 | 6-Cl | H | Benzyl | CH₃ |
| 72 | 6-Cl | H | Phenyl | CH₃ |
| 73 | 6-Cl | H | 2-Pyridyl | CH₃ |
| 74 | 6-Cl | H | 5-Cl-pyridyl-2 | CH₃ |
| 75 | 6-Cl | H | 5-CF₃-pyridyl-2 | CH₃ |
| 76 | 6-Cl | Cl | Cyclohexyl | CH₃ |
| 77 | 6-Cl | Cl | Benzyl | CH₃ |
| 78 | 6-Cl | Cl | Phenyl | CH₃ |
| 79 | 6-Cl | Cl | 2-Pyridyl | CH₃ |
| 80 | 6-Cl | Cl | 5-Cl-pyridyl-2 | CH₃ |
| 81 | 6-Cl | Cl | 5-CF₃-pyridyl-2 | CH₃ |
| 82 | 6-Cl | CH₃ | Cyclohexyl | CH₃ |
| 83 | 6-Cl | CH₃ | Benzyl | CH₃ |
| 84 | 6-Cl | CH₃ | Phenyl | CH₃ |
| 85 | 6-Cl | CH₃ | 2-Pyridyl | CH₃ |
| 86 | 6-Cl | CH₃ | 5-Cl-pyridyl-2 | CH₃ |
| 87 | 6-Cl | CH₃ | 5-CF₃-pyridyl-2 | CH₃ |
| 88 | 6-Cl | H | Cyclohexyl | C₂H₅ |
| 89 | 6-Cl | H | Benzyl | C₂H₅ |
| 90 | 6-Cl | H | Phenyl | C₂H₅ |
| 91 | 6-Cl | H | 2-Pyridyl | C₂H₅ |
| 92 | 6-Cl | H | 5-Cl-pyridyl-2 | C₂H₅ |
| 93 | 6-Cl | H | 5-CF₃-pyridyl-2 | C₂H₅ |
| 94 | 6-Cl | Cl | Cyclohexyl | C₂H₅ |
| 95 | 6-Cl | Cl | Benzyl | C₂H₅ |
| 96 | 6-Cl | Cl | Phenyl | C₂H₅ |
| 97 | 6-Cl | Cl | 2-Pyridyl | C₂H₅ |
| 98 | 6-Cl | Cl | 5-Cl-pyridyl-2 | C₂H₅ |
| 99 | 6-Cl | Cl | 5-CF₃-pyridyl-2 | C₂H₅ |
| 100 | 6-Cl | CH₃ | Cyclohexyl | C₂H₅ |
| 101 | 6-Cl | CH₃ | Benzyl | C₂H₅ |
| 102 | 6-Cl | CH₃ | Phenyl | C₂H₅ |
| 103 | 6-Cl | CH₃ | 2-Pyridyl | C₂H₅ |
| 104 | 6-Cl | CH₃ | 5-Cl-pyridyl-2 | C₂H₅ |
| 105 | 6-Cl | CH₃ | 5-CF₃-pyridyl-2 | C₂H₅ |
| 106 | 6-CH₃ | H | Cyclohexyl | CH₃ |
| 107 | 6-CH₃ | H | Benzyl | CH₃ |
| 108 | 6-CH₃ | H | Phenyl | CH₃ |
| 109 | 6-CH₃ | H | 2-Pyridyl | CH₃ |
| 110 | 6-CH₃ | H | 5-Cl-pyridyl-2 | CH₃ |
| 111 | 6-CH₃ | H | 5-CF₃-pyridyl-2 | CH₃ |
| 112 | 6-CH₃ | Cl | Cyclohexyl | CH₃ |
| 113 | 6-CH₃ | Cl | Benzyl | CH₃ |
| 114 | 6-CH₃ | Cl | Phenyl | CH₃ |
| 115 | 6-CH₃ | Cl | 2-Pyridyl | CH₃ |
| 116 | 6-CH₃ | Cl | 5-Cl-pyridyl-2 | CH₃ |
| 117 | 6-CH₃ | Cl | 5-CF₃-pyridyl-2 | CH₃ |
| 118 | 6-CH₃ | CH₃ | Cyclohexyl | CH₃ |
| 119 | 6-CH₃ | CH₃ | Benzyl | CH₃ |
| 120 | 6-CH₃ | CH₃ | Phenyl | CH₃ |
| 121 | 6-CH₃ | CH₃ | 2-Pyridyl | CH₃ |
| 122 | 6-CH₃ | CH₃ | 5-Cl-pyridyl-2 | CH₃ |
| 123 | 6-CH₃ | CH₃ | 5-CF₃-pyridyl-2 | CH₃ |
| 124 | 6-CH₃ | H | Cyclohexyl | C₂H₅ |
| 125 | 6-CH₃ | H | Benzyl | C₂H₅ |
| 126 | 6-CH₃ | H | Phenyl | C₂H₅ |
| 127 | 6-CH₃ | H | 2-Pyridyl | C₂H₅ |
| 128 | 6-CH₃ | H | 5-Cl-pyridyl-2 | C₂H₅ |
| 129 | 6-CH₃ | H | 5-CF₃-pyridyl-2 | C₂H₅ |
| 130 | 6-CH₃ | Cl | Cyclohexyl | C₂H₅ |
| 131 | 6-CH₃ | Cl | Benzyl | C₂H₅ |
| 132 | 6-CH₃ | Cl | Phenyl | C₂H₅ |
| 133 | 6-CH₃ | Cl | 2-Pyridyl | C₂H₅ |
| 134 | 6-CH₃ | Cl | 5-Cl-pyridyl-2 | C₂H₅ |
| 135 | 6-CH₃ | Cl | 5-CF₃-pyridyl-2 | C₂H₅ |
| 136 | 6-CH₃ | CH₃ | Cyclohexyl | C₂H₅ |
| 137 | 6-CH₃ | CH₃ | Benzyl | C₂H₅ |
| 138 | 6-CH₃ | CH₃ | Phenyl | C₂H₅ |
| 139 | 6-CH₃ | CH₃ | 2-Pyridyl | C₂H₅ |
| 140 | 6-CH₃ | CH₃ | 5-Cl-pyridyl-2 | C₂H₅ |
| 141 | 6-CH₃ | CH₃ | 5-CF₃-pyridyl-2 | C₂H₅ |

Die erfindungsgemäßen Verbindungen der Formel I eignen sich zur Bekämpfung von Schadpilzen und von tierischen Schädlingen aus der Klasse der Insekten, Spinnentiere und Nematoden. Sie können im Pflanzenschutz sowie auf dem Hygiene-, Vorratsschutz- und Veterinärsektor als Fungizide und Schädlingsbekämpfungsmittel eingesetzt werden.

Zu den schädlichen Insekten gehören:
aus der Ordnung der Schmetterlinge (Lepidoptera) beispielsweise Adoxophyes orana, Agrotis ypsilon, Agrotis segetum, Alabama argillacea, Anticarsia gemmatalis, Argyresthia conjugella, Autographa gamma, Cacoecia murinana, Capua reticulana, Choristoneura fumiferana, Chilo partellus, Choristoneura occidentalis, Cirphis unipuncta, Cnaphalocrocis medinalis, Crocidolomia binotalis, Cydia pomonella, Dendrolimus pini, Diaphania nitidalis, Diatraea grandiosella, Earias insulana, Elasmopalpus lignosellus, Eupoecilia ambiguella, Feltia subterranea, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera scitella, Lithocolletis blancardella, Lobesia botrana, Loxostege sticticalis, Lymantria dispar, Lymantria monacha, Lyonetia clerkella, Manduca sexta, Malacosoma neustria, Mamestra brassicae, Mocis repanda, Operophthera brumata, Orgyia pseudotsugata, Ostrinia nubilalis, Pandemis heparana, Panolis flammea, Pectinophora gossypiella, Phthorimaea operculella, Phyllocnistis citrella, Pieris brassicae, Plathypena scabra, Platynota stultana, Plutella xylostella, Prays citri, Prays oleae, Prodenia sunia, Prodenia ornithogalli, Pseudoplusia includens, Rhyacionia frustrana, Scrobipalpula absoluta, Sesamia inferens, Sparganothis pilleriana, Spodoptera frugiperda, Spodoptera littoralis, Spodoptera litura, Syllepta derogata, Synanthedon myopaeformis, Thaumatopoea pityocampa, Tortrix viridana, Trichoplusia ni, Tryporyza incertulas, Zeiraphera canadensis, ferner Galleria mellonella und Sitotroga cerealella, Ephestia cautella, Tineola bisselliella;
aus der Ordnung der Käfer (Coleoptera) beispielsweise Agriotes lineatus, Agriotes obscurus, Anthonomus grandis, Anthonomus pomorum, Apion vorax, Atomaria linearis, Blastophagus piniperda, Cassida nebulosa, Cerotoma trifurcata, Ceuthorhynchus assimilis, Ceuthorhynchus napi, Chaetocnema tibialis, Conoderus vespertinus, Crioceris asparagi, Dendroctonus refipennis, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllopertha horticola, Phyllophaga sp., Phyllotreta chrysocephala, Phyllotreta nemorum, Phyllotreta striolata, Popillia japonica, Psylliodes napi, Scolytus intricatus, Sitona lineatus, ferner Bruchus rufimanus, Bruchus pisorum, Bruchus lentis, Sitophilus granaria, Lasioderma serricorne, Oryzaephilus surinamensis, Rhyzopertha dominica, Sitophilus oryzae, Tribolium castaneum, Trogoderma granarium, Zabrotes subfasciatus;
aus der Ordnung der Zweiflügler (Diptera) beispielsweise Anastrepha ludens, Ceratitis capitata, Contarinia sorghicola, Dacus cucurbitae, Dacus oleae, Dasineura brassicae, Delia coarctata, Delia radicum, Hydrellia griseola, Hylemyia platura, Liriomyza sativae, Liriomyza trifolii, Mayetiola destructor, Orseolia oryzae, Oscinella frit, Pegomya hyoscyami, Phorbia antiqua, Phorbia brassicae, Phorbia coarctata, Rhagoletis cerasi, Rhagoletis pomonella, Tipula oleracea, Tipula paludosa, ferner Aedes aegypti, Aedes vexans, Anopheles maculipennis, Chrysomya bezziana, Chrysomya hominivorax, Chrysomya macellaria, Cordylobia anthropophaga, Culex pipiens, Fannia canicularis, Gasterophilus intestinalis, Glossina morsitans, Haematobia irritans, Haplodiplosis equestris, Hypoderma lineata, Lucilia caprina, Lucilia cuprina, Lucilia sericata, Musca domestica, Muscina stabulans, Oestrus ovis, Tabanus bovinus, Simulium damnosum;
aus der Ordnung der Thripse (Thysanoptera) beispielsweise Frankliniella fusca, Frankliniella occidentalis, Frankliniella tritici, Haplothrips tritici, Scirtothrips citri, Thrips oryzae, Thrips palmi, Thrips tabaci;
aus der Ordnung der Hautflügler (Hymenoptera) beispielsweise Athalia rosae, Atta cephalotes, Atta sexdens, Atta texana, Hoplocampa minuta, Hoplocampa testudinea, Iridomyrmes humilis, Iridomyrmex purpureus, Monomorium pharaonis, Solenopsis geminata, Solenopsis invicta, Solenopsis richteri;
aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus hesperus, Lygus lineolaris, Lygus pratensis, Nezara viridula, Piesma quadrata, Solubea insularis, Thyanta perditor;
aus der Ordnung der Pflanzensauger (Homoptera) beispielsweise Acyrthosiphon onobrychis, Acyrthosiphon pisum, Adelges laricis, Aonidiella aurantii, Aphidula nasturtii, Aphis fabae, Aphis gossypii, Aphis pomi, Aulacorthum solani, Bemisia tabaci, Brachycaudus cardui, Brevicoryne brassicae, Dalbulus maidis, Dreyfusia nordmannianae, Dreyfusia piceae, Dysaphis radicola, Empoasca fabae, Eriosoma lanigerum, Laodelphax striatella, Macrosiphum avenae, Macrosiphum euphorbiae, Macrosiphon rosae, Megoura viciae, Metopolophium dirhodum, Myzus persicae, Myzus cerasi, Nephotettix cincticeps, Nilaparvata lugens, Perkinsiella saccharicida, Phorodon humuli, Planococcus citri, Psylla mali, Psylla piri, Psylla pyricol, Quadraspidiotus perniciosus, Rhopalosiphum maidis, Saissetia oleae, Schizaphis graminum, Selenaspidus articulatus, Sitobion avenae, Sogatella furcifera, Toxoptera citricida, Trialeurodes abutilonea, Trialeurodes vaporariorum, Viteus vitifolii;
aus der Ordnung der Termiten (Isoptera) beispielsweise Calotermes flavicollis, Leucotermes flavipes, Macrotermes subhyalinus, Odontotermes formosanus, Reticulitermes lucifugus, Termes natalensis;
aus der Ordnung der Geradflügler (Orthoptera) beispielsweise Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Schistocerca americana, Schistocerca peregrina, Stauronotus maroccanus, Schistocerca gregaria, ferner Acheta domestica, Blatta orientalis, Blattella germanica, Periplaneta americana;
aus der Ordnung der Arachnoidea beispielsweise phytophage Milben wie Aculops lycopersicae, Aculops pelekassi, Aculus schlechtendali, Brevipalpus phoenicis, Bryobia praetiosa, Eotetranychus carpini, Eutetranychus banksii, Eriophyes sheldoni, Oligonychus pratensis, Panonychus ulmi, Panonychus citri, Phyllocoptruta oleivora, Polyphagotarsonemus latus, Tarsonemus pallidus, Tetranychus cinnabarinus, Tetranychus kanzawai, Tetranchus pacificus, Tetranychus urticae, Zecken wie Amblyomma americanum, Amblyomma variegatum, Argas persicus, Boophilus annulatus, Boophilus decoloratus, Boophilus microplus, Dermacentor silvarum, Hyalomma truncatum, Ixodes ricinus, Ixodes rubicundus, Ornithodorus moubata, Otobius megnini, Rhipicephalus appendiculatus und Rhipicephalus evertsi sowie tierparasitische Milben wie Dermanyssus gallinae, Psoroptes ovis und Sarcoptes scabiei;
aus der Klasse der Nematoden beispielsweise Wurzelgallennematoden, z.B. Meloidogyne hapla, Meloidogyne incognita, Meloidogyne javanica, zystenbildende Nematoden, z.B. Globodera pallida, Globodera rostochiensis, Heterodera avenae, Heterodera glycines, Heterodera schachtii, migratorische Endoparasiten und semi-endoparasitische Nematoden, z.B. Heliocotylenchus multicinctus, Hirschmanniella oryzae, Hoplolaimus spp, Pratylenchus brachyurus, Pratylenchus fallax, Pratylenchus penetrans, Pratylenchus vulnus, Radopholus similis, Rotylenchus reniformis, Scutellonema bradys, Tylenchulus semipenetrans, Stock- und Blattnematoden z.B. Anguina tritici, Aphelenchoides besseyi, Ditylenchus angustus, Ditylenchus dipsaci, Virusvektoren, z.B. Longidorus spp, Trichodorus christei, Trichodorus viruliferus, Xiphinema index, Xiphinema mediterraneum.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Als Fungizide sind die Verbindungen der Formel I z.T. systemisch wirksam. Sie können als Blatt- und Bodenfungizide gegen ein breites Spektrum von pflanzenpathogenen Pilzen, insbesondere aus der Klasse der Ascomyceten, Deuteromyceten, Phycomyceten und Basidiomyceten eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Rasen, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sich die Verbindungen I zur Bekämpfung folgender Pflanzenkrankheiten:
* Erysiphe graminis (echter Mehltau) in Getreide,
* Erysiphe cichoracearum und Sphaerotheca fuliginea an Kürbisgewächsen,
* Podosphaera leucotricha an Äpfeln,
* Uncinula necator an Reben,
* Puccinia-Arten an Getreide,
* Rhizoctonia-Arten an Baumwolle und Rasen,
* Ustilago-Arten an Getreide und Zuckerrohr,
* Venturia inaequalis (Schorf) an Äpfeln,
* Helminthosporium-Arten an Getreide,
* Septoria nodorum an Weizen,
* Botrytis cinerea (Grauschimmel) an Erdbeeren, Reben,
* Cercospora arachidicola an Erdnüssen,
* Pseudocercosporella herpotrichoides an Weizen, Gerste,
* Pyricularia oryzae an Reis,
* Phytophthora infestans an Kartoffeln und Tomaten,
* Fusarium- und Verticillium-Arten an verschiedenen Pflanzen,
* Plasmopara viticola an Reben,
* Alternaria-Arten an Gemüse und Obst.

Die neuen Verbindungen können auch im Materialschutz (Holzschutz) eingesetzt werden, z.B. gegen Paecilomyces variotii.

Sie können in die üblichen Formulierungen übergeführt werden, wie Lösungen, Emulsionen, Suspensionen, Stäube, Pulver, Pasten oder Granulate. Die Anwendungsformen richten sich dabei nach dem jeweiligen Verwendungszweck; sie sollten in jedem Fall möglichst die feinste Verteilung der Wirkstoffe gewährleisten.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können.

Als Hilfsstoffe kommen dafür im wesentlichen in Betracht:
- Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser;
- Trägerstoffe wie natürliche Gesteinsmehle (z.B. Kaoline, Tonerden, Talkum, Kreide) und synthetische Gesteinsmehle (z.B. hochdisperse Kieselsäure, Silikate);
- Emulgiermittel wie nichtionogene und anionische Emulgatoren (z.B. Polyoxyethylen-Fettalkohol-Ether, Alkylsulfonate und Arylsulfonate) und
- Dispergiermittel wie Ligninsulfit-Ablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden.

Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe. Die Wirkstoffkonzentrationen in den anwendungsfertigen Zubereitungen können in größeren Bereichen variiert werden.

Ganz allgemein enthalten die Mittel zwischen 0,0001 und 95 Gew.-% Wirkstoff.

Formulierungen mit mehr als 95 Gew.-% Wirkstoff können mit gutem Erfolg im Ultra-Low-Volume-Verfahren (ULV) ausgebracht werden, wobei sogar der Wirkstoff ohne Zusätze verwendet werden kann.

Für die Anwendung als Fungizide empfehlen sich Konzentrationen zwischen 0,01 und 95 Gew.%, vorzugsweise zwischen 0,5 und 90 Gew.%, Wirkstoff. Für die Anwendung als Insektizide kommen Formulierungen mit 0,0001 bis 10 Gew.%, vorzugsweise 0,01 bis 1 Gew.% Wirkstoff, in Betracht.

Die Wirkstoffe werden normalerweise in einer Reinheit von 90 % bis 100 %, vorzugsweise 95 % bis 100 % (nach NMR-Spektrum) eingesetzt.

Beispiele für solche Zubereitungen sind:
I. eine Lösung aus 90 Gew.-Teilen einer erfindungsgemäßen Verbindung I und 10 Gew.-Teilen N-Methyl-a-pyrrolidon, die zur Anwendung in Form kleinster Tropfen geeignet ist;
II. eine Lösung von 20 Gew.-Teilen einer erfindungsgemäßen Verbindung I in einer Mischung aus 80 Gew.-Teilen alkyliertem Benzol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes von 40 Mol Ethylen oxid an 1 Mol Ricinusöl; durch feines Verteilen der Formulierung in Wasser erhält man eine Dispersion.
III. eine Lösung von 20 Gew.-Teilen einer erfindungsgemäßen Verbindung I in einer Mischung aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl; durch feines Verteilen der Formulierung in Wasser erhält man eine Dispersion.
IV. eine wäßrige Dispersion aus 20 Gew.-Teilen einer erfindungsgemäßen Verbindung I, in einer Mischung aus 25 Gew.-Teilen Cyclohexanon, 65 Gew.-Teilen einer Mineralölfraktion vom Siedepunkt 210 bis 280 °C und 10 Gew.-Teilen des Anlagerungsproduktes von 40 mol Ethylenoxid an 1 mol Ricinusöl; durch feines Verteilen der Formulierung in Wasser erhält man eine Dispersion.
V. eine in einer Hammermühle vermahlene Mischung aus 20 Gew.-Teilen einer erfindungsgemäßen Verbindung I, 3 Gew.-Teilen des Natriumsalzes der Diisobutylnaphtalin-α-sulfonsäure, 17 Gew.-Teilen des Natriumsalzes einer Ligninsulfonsäure aus einer Sulfitablauge und 60 Gew.-Teilen pulverförmigem Kieselsäuregel; durch feines Verteilen der Mischung in Wasser erhält man eine Spritzbrühe;
VI. eine innige Mischung aus 3 Gew.-Teilen einer erfindungsgemäßen Verbindung I und 97 Gew.-Teilen feinteiligem Kaolin; dieses Stäubemittel enthält 3 Gew.-% Wirkstoff;
VII. eine innige Mischung aus 30 Gew.-Teilen einer erfindungsgemäßen Verbindung I, 92 Gew.-Teilen pulverförmigem Kieselsäuregel und 8 Gew.Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen einer erfindungsgemäßen Verbindung I, 10 Gew.-Teilen des Natriumsalzes eines Phenosulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen einer erfindungsgemäßen Verbindung I, 2 Gew.-Teilen des Calciumsalzes der Dodecylbenzolsulfonsäure, 8 Gew.-Teilen Fettalkohol-polyglykol-ether, 2 Gew.-Teilen des Natriumsalzes eines Phenolsulfonsäure-harnstoff-formaldehyd-Kondensates und 68 Gew.-Teilen eines paraffinischen Mineralöls;
X. eine in einer Hammermühle vermahlene Mischung aus 10 Gew.-Teilen einer erfindungsgemäßen Verbindung I, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.-Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Verbindungen I werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Die Aufwandmengen liegen je nach Art des gewünschten Effektes zwischen 0,02 und 3 kg Wirkstoff pro ha, vorzugsweise bei 0,1 bis 1 kg/ha.

Bei der Saatgutbehandlung werden im allgemeinen Wirkstoffmengen von 0,001 bis 50 g, vorzugsweise 0,01 bis 10 g je Kilogramm Saatgut benötigt.

Die Aufwandmenge an Wirkstoff für die Bekämpfung von Schädlingen beträgt unter Freilandbedingungen 0,02 bis 10, vorzugsweise 0,1 bis 2,0 kg/ha Wirkstoff.

Die Verbindungen I, allein oder in Kombination mit Herbiziden oder Fungiziden, können auch noch mit weiteren Pflanzenschutzmitteln gemischt gemeinsam ausgebracht werden, beispielsweise mit Wachstumsregulatoren oder mit Mitteln zur Bekämpfung von Schädlingen oder Bakterien. Von Interesse ist ferner die Mischbarkeit mit Düngemitteln oder mit Mineralsalzlösungen, welche zur Behebung von Ernährungs- und Spurenelementmängeln eingesetzt werden.

Die Pflanzenschutz- und Düngemittel können zu den erfindungsgemäßen Mitteln im Gewichtsverhältnis 1:10 bis 10:1 zugesetzt werden, gegebenenfalls auch erst unmittelbar vor der Anwendung (Tankmix). Beim Vermischen mit Fungiziden oder Insektiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'propylen-bis-dithiocarbamat), N,N'-Polypropylen-bis-(thiocarbamoyl)-disulfid; Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthals°ure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-β-[bis-(dimethylamino)-phosphinyl]-3-phenyl-1,2,4-triazol, 2,3-Dicyano-1,4-dithioanthrachinon, 2-Thio-1,3-dithiolo-β-[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid, N-Dichlorfluormethylthio-N',N'-dimethyl-N-phenylschwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iodbenzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclodedecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-l-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,
sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl) -alanin-methylester, N-(2,6-Dimethylphenyl)-N-chloracetylD,L-2-aminobutyrolacton, DL-N-(2,6-Dimethylphenyl)-N-(phenylacetyl) -alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl] -1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in der anschließenden Tabelle mit physikalischen Daten aufgeführt.
1. N-(2-(N'-(o-Chlorphenyl)-5'-methyl-triazolyl-3'-oxymethyl)-phenyl)-N-methoxy-carbaminsäuremethylester (Tabelle, Nr. 11)
   Eine Mischung von 3,3 g (Reinheit ca. 80 %ig, ≙ 10 mmol) N-(2-Brommethylphenyl)-N-methoxy-carbaminsäuremethylester (WO 93/15046), 2,1 g (10 mmol) N-(o-Chlorphenyl)-3-hydroxy-5-methyltriazol und 2 g (15 mmol) K₂CO₃ in 20 ml DMF wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden mit Wasser extrahiert, über MgSO₄ getrocknet und eingeengt. Der Rückstand wird säulenchromatographisch mit Cyclohexan/Essigsäureethylester-Gemischen gereinigt. Man erhält 1,1 g (27 %) der Titelverbindung als gelbes Öl.
   ¹H-NMR(CDCl₃; δ in ppm): 7,7 (m, 1H, Phenyl); 7,55 (m, 1H, Phenyl); 7,4 (m, 6H, Phenyl); 5,35 (s, 2H, OCH₂); 3,72, 3,77 (2s, je 3H, 2 x OCH₃); 2,25, (s, 3H, CH₃)
2. N-(2-(N'-Phenyl-5'chlor-triazolyl-3'-oxymethyl)-phenyl)-N-methoxy-carbaminsäuremethylester (Tabelle, Nr. 15)
   Eine Mischung von 3,3 g (Reinheit ca. 80%ig; 10 mmol N-(2-Brommethylphenyl)-N-methoxy-carbaminsäuremethylester (WO 93/15046), 2 g (10 mmol) N-Phenyl-5-chlor-3-hydroxytriazol und 1,8 g (13 mmol) K₂CO₃ in 20 ml DMF wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden mit Wasser gewaschen, über MgSO₄ getrocknet und eingeengt. Der Rückstand wird säulenchromatographisch mit Cyclohexan/Essigester-Gemischen gereinigt. Man erhält 2,7 g (69 %) der Titelverbindung als gelbes Öl.
3. N-(2-N'-Pyridyl-2"-triazoyl-3'-oxymethyl)-phenyl)-N-methoxycarbaminsäuremethylester (Tabelle, Nr. 40)
   Eine Mischung von 2,7 g (Reinheit ca. 80%ig; 8 mmol N-(2-Brommethylphenyl)-N-methoxy-carbaminsäuremethylester (WO 93/15046), 1,7 g (8 mmol) N-(Pyridyl-2')-3-hydroxytriazol und 1,7 g (12 mmol) K₂CO₃ in 20 ml DMF wird über Nacht bei Raumtemperatur gerührt. Anschließend verdünnt man die Reaktionsmischung mit Wasser und extrahiert die wäßrige Phase dreimal mit Methyl-t-butylether. Die vereinigten organischen Phasen werden mit Wasser extrahiert, über MgSO₄ getrocknet und eingeengt. Der Rückstand wird säulenchromatographisch mit Cyclohexan/Essigester-Gemischen gereinigt. Man erhält 1,4 g (49 %) der Titelverbindung als farblosen Festkörper (Fp = 89°C) .
   ¹H-NMR (CDCl₃; δ in ppm) : 8,9 (s, 1H, Triazolyl); 8,4 (m, 1H, (Het)aryl); 7,8 (m, 3H, (Het)aryl); 7,4 (m, 3H, (Het)aryl); 7,25 (m, 1H, (Het)aryl); 5,45 (s, 2H, OCH₂); 3,8, 4,75 (2s, je 3H, 2 x OCH₃)

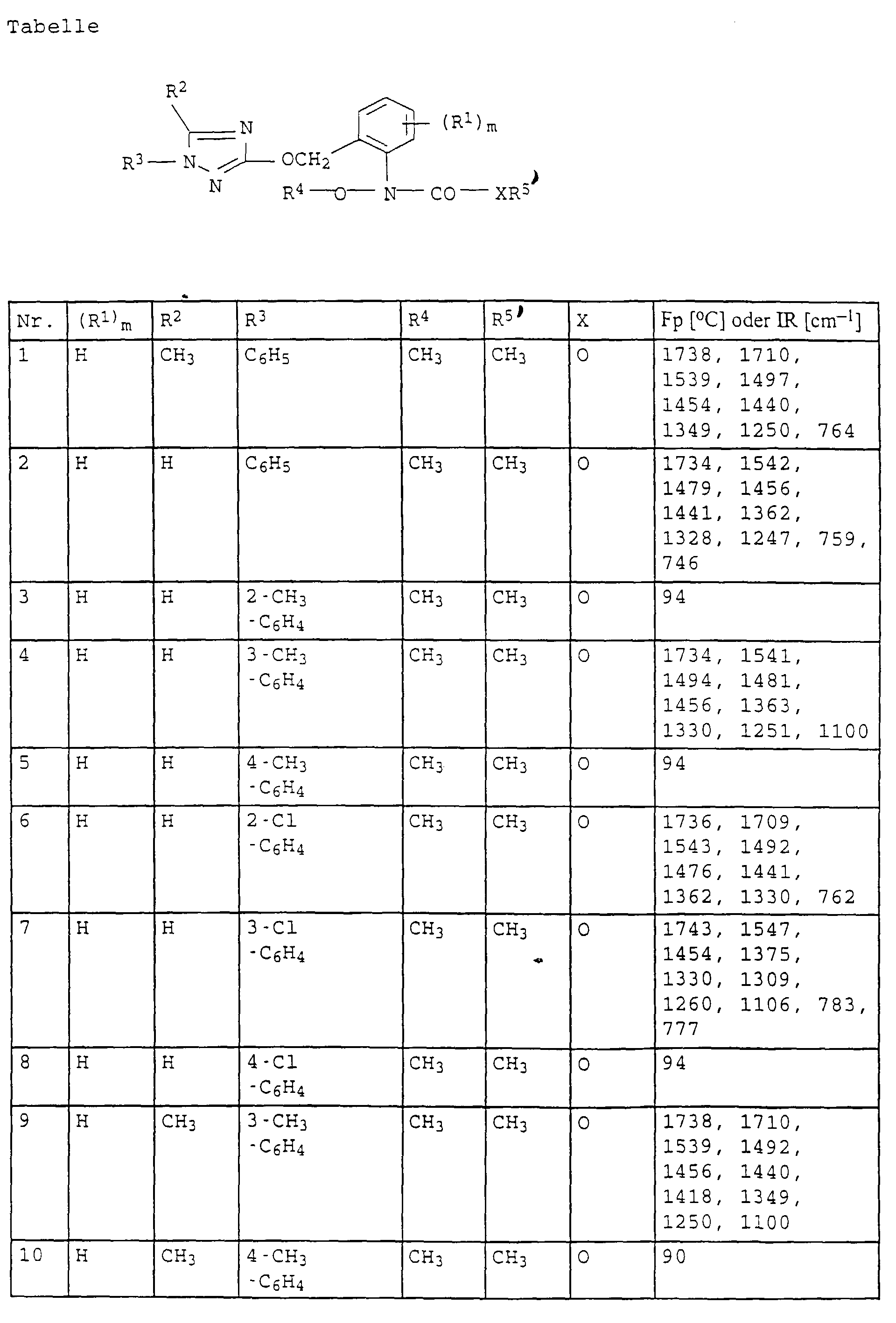

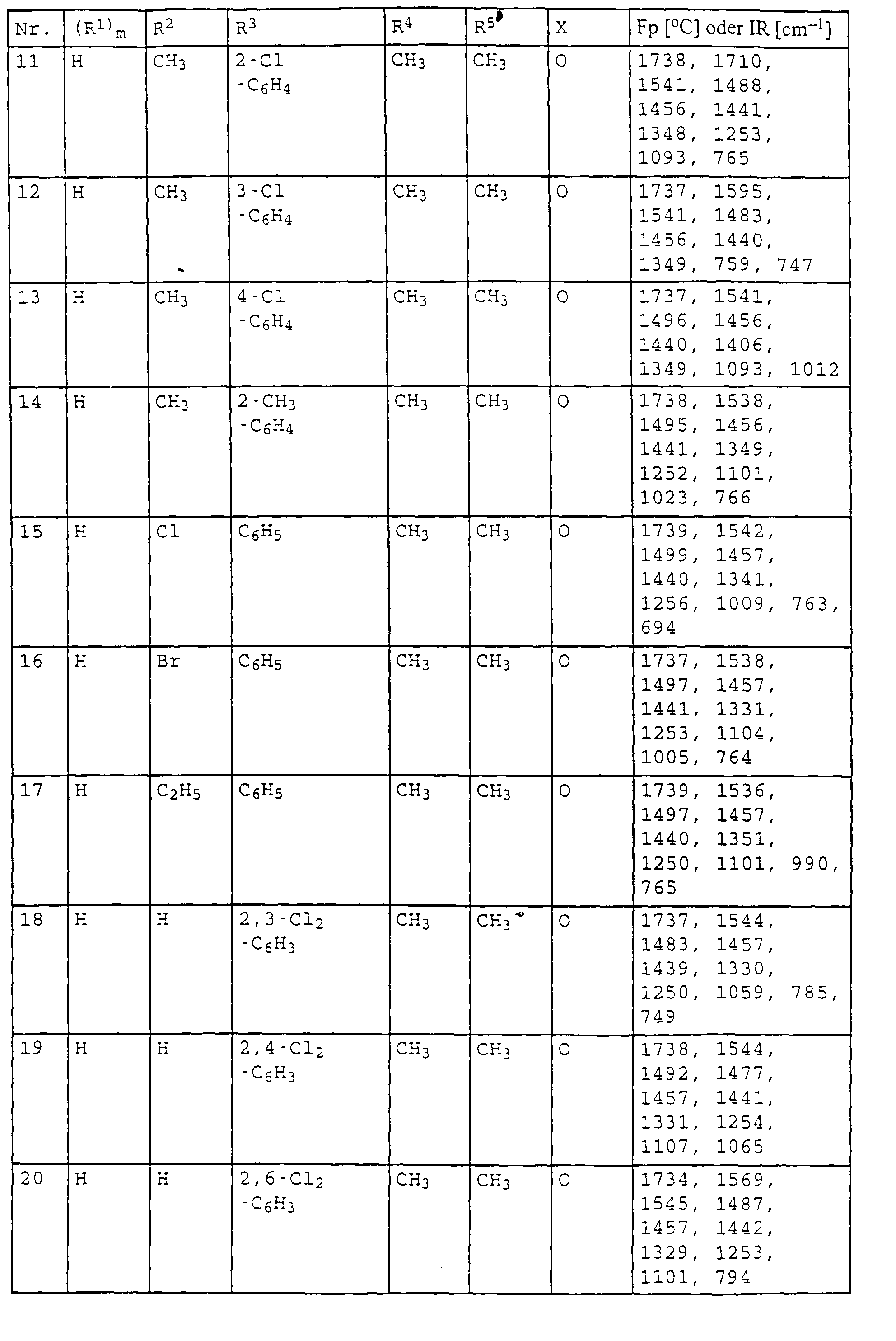

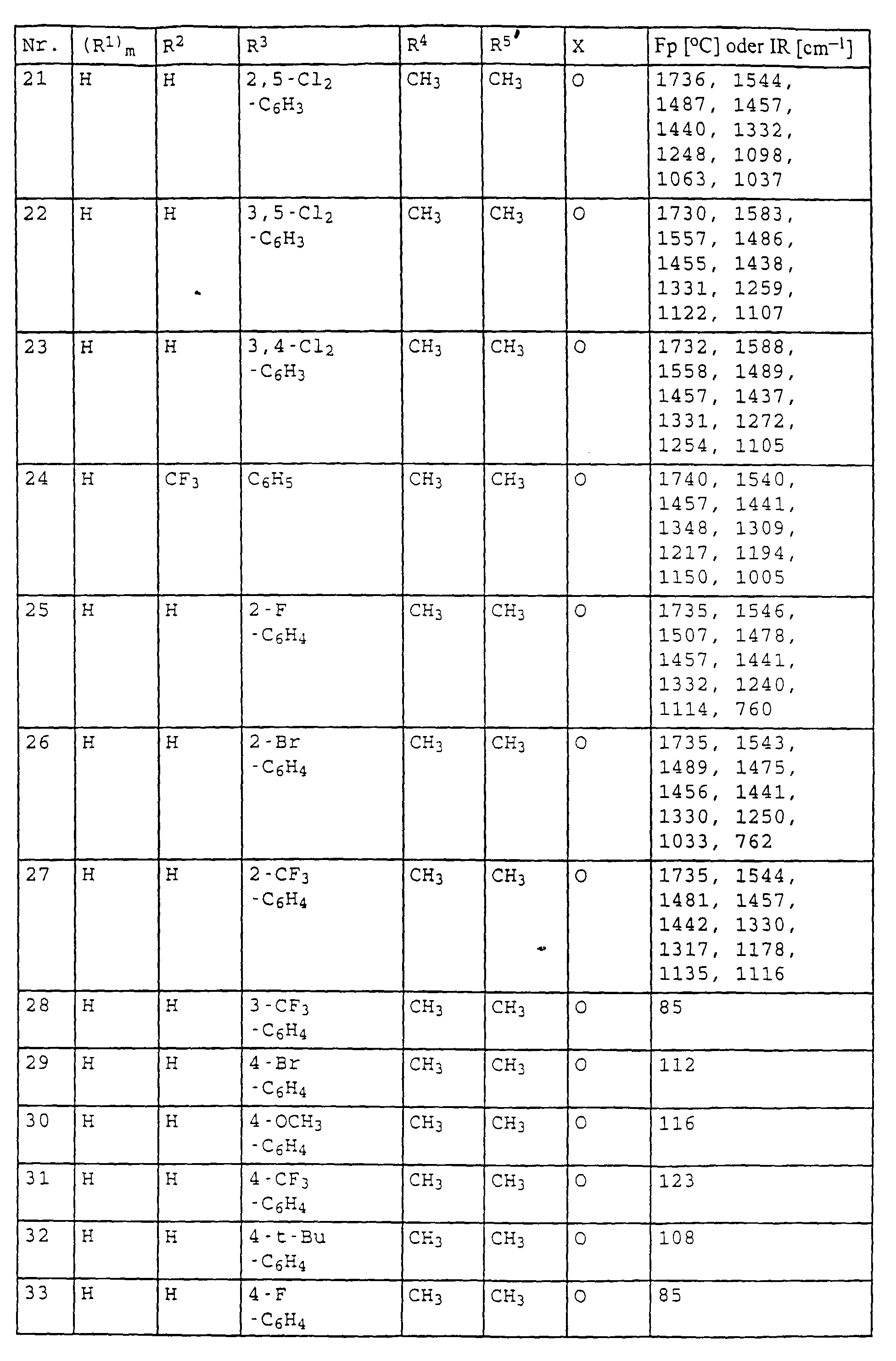

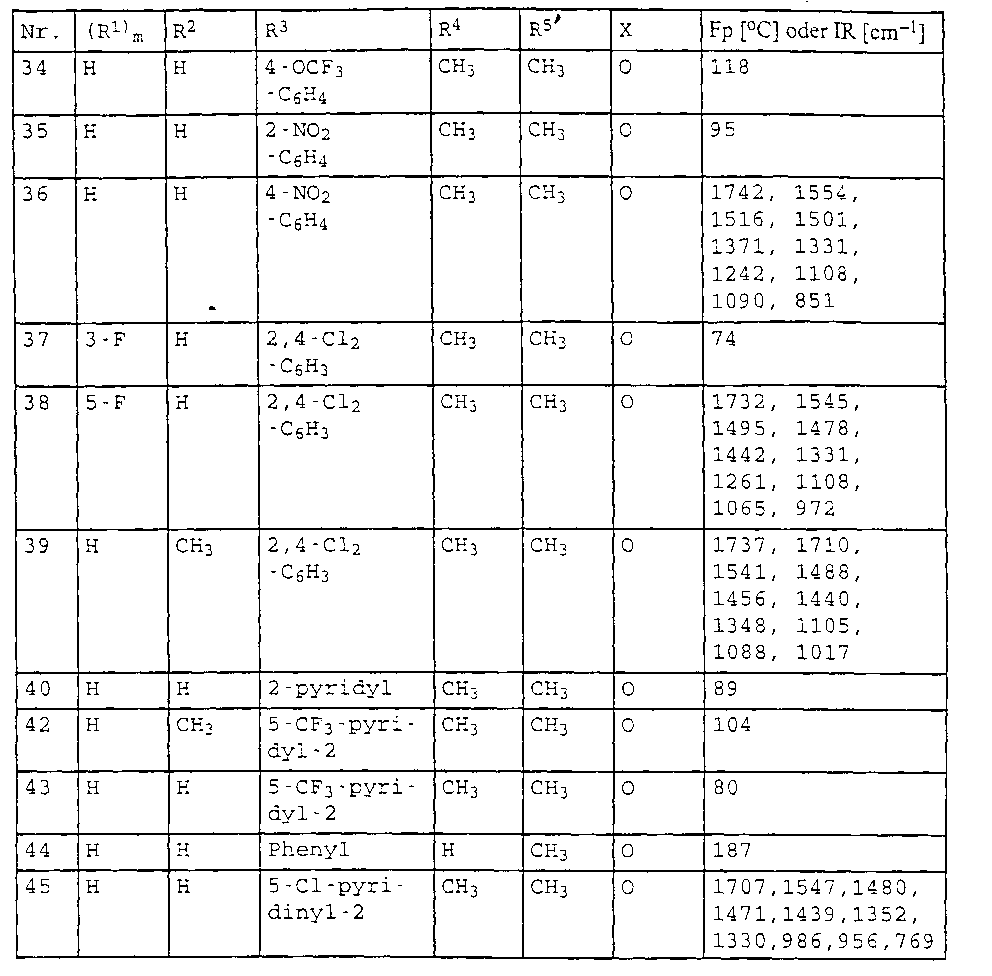

### Beispiele zur Wirkung gegen Schadpilze

Die fungizide Wirkung der Verbindungen der Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### Wirksamkeit gegen Puccinia recondita

Blätter von Weizensämlingen (Sorte "Kanzler") wurden mit Sporen des Braunrosts *(Puccinia recondita)* bestäubt. Die so behandelten Pflanzen wurden 24 h bei 20-22°C und einer relativen Luftfeuchtigkeit von 90-95% inkubiert und anschließend mit der wäßrigen Wirkstoffaufbereitung (250 ppm Wirkstoff) behandelt. Nach weiteren 8 Tagen bei 20-22°C und 65-70% relativer Luftfeuchtigkeit wurde das Ausmaß der Pilzentwicklung ermittlelt. Die Auswertung erfolgte visuell.

In diesem Test zeigten die mit den erfindungsgemäßen Verbindungen 1, 2, 4-10, 12, 13, 15, 17-19, 21, 23-26, 28-31, 33, 37, 38, 42, 43 und 45 behandelten Pflanzen einen Befall von 10% und weniger. Die unbehandelten Pflanzen waren zu 70% befallen.

### Wirksamkeit gegen Botrytis cinerea

Paprikasämlinge (Sorte: "Neusiedler Ideal Elite") mit 4-5 Blättern wurden mit der Wirkstoffaufbereitung (Aufwandmenge: 500 ppm) tropfnaß gespritzt. Nach dem Abtrocknen wurden die Pflanzen mit einer Konidienaufschwemmung des Pilzes *Botrytis cinerea* besprüht und 5 Tage bei 22-24°C bei hoher Luftfeuchtigkeit bewahrt. Die Auswertung erfolgte visuell.

In diesem Test zeigten die mit der erfindungsgemäßen Verbindung Nr. 2 behandelten Pflanzen 5% Befall, während die unbehandelten Pflanzen zu 80% befallen waren.

### Wirksamkeit gegen Pyricularia oryzae

Reiskeimlinge (Sorte: "Tai Nong 67") wurden mit der Wirkstoffaufbereitung (Aufwandmenge 250 ppm) tropfnaß gespritzt. Nach 24 Stunden wurden die Pflanzen mit einer wäßrigen Sporensuspension des Pilzes *Pyricularia oryzae* besprüht und 6 Tage bei 22-24°C bei einer relativen Luftfeuchtigkeit von 95-99 % bewahrt. Die Beurteilung erfolgte visuell.

In einem entsprechenden Test zeigten die mit 250 ppm den erfindungsgemäßen Verbindungen 2, 6-8, 13, 15, 17-19, 21, 24-38 und 42-45 einen Befall von 5% und weniger; die unbehandelten Pflanzen waren zu 70% befallen.

### Wirksamkeit gegen Fusarium culmorum

Primär-Blätter von Weizensämlingen (Sorte "Kanzler") wurden mit der Wirkstoffaufbereitung (Aufwandmenge 500 ppm) tropfnaß gespritzt. Am folgenden Tag wurden die Pflanzen mit einer Sporensuspension von Fusarium culorum infiziert. Die so behandelten Pflanzen wurden 6 Tage bei 22-24°C und einer relativen Luftfeuchtigkeit von >90% inkubiert. Die Auswertung erfolgte visuell.

In diesem Test zeigten die mit der erfindungsgemäßen Verbindung Nr. 2 behandelten Pflanzen 5% Befall, während die unbehandelten Pflanzen zu 60% befallen waren.

### Beispiele zur Wirkung gegen tierische Schädlinge

Die Wirkung der Verbindungen der allgemeinen Formel I gegen tierische Schädlinge ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

## Patentansprüche

1. 2-[1',2',4'-Triazol-3'-yloxymethylen]-anilide der Formel I 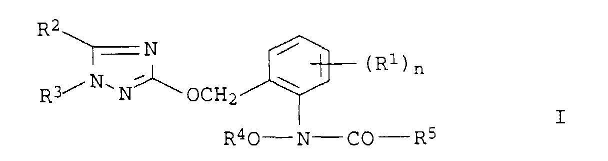 in der der Index und die Substituenten die folgende Bedeutung haben:
n 0, 1, 2, 3 oder 4, wobei die Substituenten R¹ verschieden sein können, wenn n größer als 1 ist;
R¹ Halogen, C₁-C₄-Alkyl, C₁-C₂-Halogenalkyl, C₁-C₄-Alkoxy oder C₁-C₂-Halogenalkoxy;
R² Wasserstoff, Nitro, Cyano, Halogen, C₁-C₄-Alkyl, C₁-C₄-Halogenalkyl, C₁-C₄-Alkoxy, C₁-C₄-Alkylthio oder C₁-C₄-Alkoxycarbonyl;
R³ C₁-C₄-Alkyl oder C₃-C₆-Cycloalkyl
Phenyl oder Benzyl, wobei der Phenylteil partiell oder vollständig halogeniert sein kann und/oder
- ein bis drei der folgenden Reste: Cyano, Nitro, C₁-C₆-Alkyl, C₁-C₄-Halogenalkyl, C₁-C₄-Alkoxy, C₁-C₄-Halogenalkoxy, C₁-C₄-Alkoxy-C₁-C₄-alkyl, C₃-C₆-Cycloalkyl, C₁-C₄-Alkylcarbonyl, C₁-C₄-Alkoxycarbonyl, Phenyl, Phenoxy und Phenyl-C₁-C₄-alkoxy, wobei die Phenylringe ihrerseits partiell oder vollständig halogeniert sein können und/oder ein bis drei der folgenden Reste tragen können: Cyano, Nitro, C₁-C₄-Alkyl, C₁-C₂-Halogenalkyl, C₁-C₄-Alkoxy, C₁-C₂-Halogenalkoxy, C₃-C₆-Cycloalkyl, C₁-C₄-Alkylcarbonyl oder C₁-C₄-Alkoxycarbonyl, und/oder
- eine Gruppe CR'=NOR", in der R' Wasserstoff oder C₁-C₄-Alkyl bedeutet und R" für C₁-C₆-Alkyl steht, und/oder
- zwei benachbarte C-Atome des Phenylrings über eine Oxy-C₁-C₃-alkoxy-Brücke oder eine Oxy-C₁-C₃-halogenalkoxy-Brücke
tragen kann,
Pyridyl oder Pyrimidyl, wobei der heteroaromatische Ring partiell oder vollständig halogeniert sein kann und/oder ein bis drei der folgenden Reste tragen kann: Cyano, Nitro, C₁-C₄-Alkyl, C₁-C₂-Halogenalkyl, C₁-C₄-Alkoxy, C₁-C₂-Halogenalkoxy, C₃-C₆-Cycloalkyl, C₁-C₄-Alkylcarbonyl oder C₁-C₄-Alkoxycarbonyl;
R⁴ Wasserstoff, C₁-C₄-Alkyl oder C₁-C₂-Halogenalkyl;
R⁵ Methyl, Ethyl, Cyclopropyl, Methoxy oder Methylamino.

2. Verfahren zur Herstellung der Verbindungen der Formel I gemäß Anspruch 1, in denen R⁴ Wasserstoff bedeutet und R⁵ für Methyl, Ethyl, Cyclopropyl oder Methoxy steht, dadurch gekennzeichnet, daß man ein Benzylderivat der Formel II, 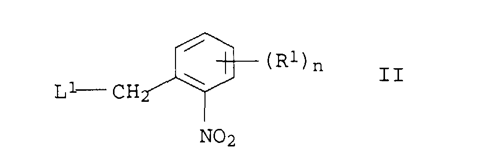 in der L¹ eine nucleophil austauschbare Gruppe bedeutet, in Gegenwart einer Base mit einem 3-Hydroxytriazol der Formel III 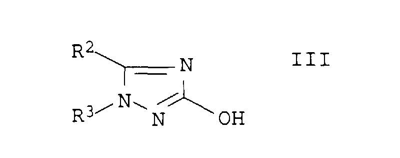 in das entsprechende 2-[1',2',4'-Triazol-3'-yloxymethylen]-nitrobenzol der Formel IV 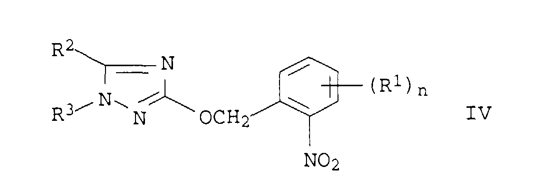 überführt, IV anschließend zum N-Hydroxylanilin der Formel Va 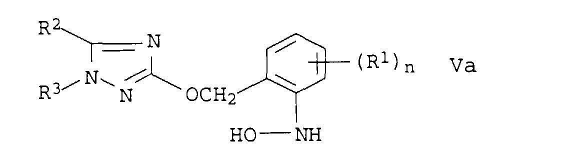 reduziert und Va mit einer Carbonylverbindung der Formel VI
L²―CO―R⁵ VI
in der L² eine nucleophil austauschbare Gruppe bedeutet, in I umwandelt.

3. Verfahren zur Herstellung der Verbindungen der Formel I gemäß Anspruch 1, in denen R⁴ nicht Wasserstoff bedeutet und R⁵ für Methyl, Ethyl, Cyclopropyl oder Methoxy steht, dadurch gekennzeichnet, daß man ein Benzylderivat der Formel IIa 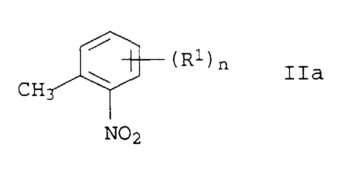 zunächst zum entsprechenden Hydroxyanilin der Formel Vb 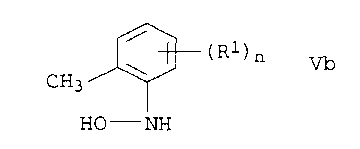 reduziert, Vb mit einer Carbonylverbindung der in Anspruch 2 definierten Formel VI in das entsprechende Anilid der Formel VII 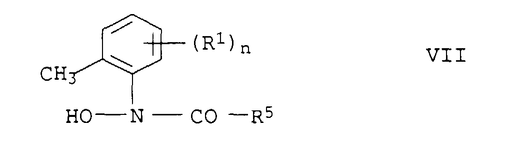 überführt, VII anschließend mit einer Verbindung VIII
L³-R⁴ VIII
in der L³ eine nucleophil austauschbare Gruppe bedeutet und R⁴ nicht für Wasserstoff steht, in das Amid der Formel IX 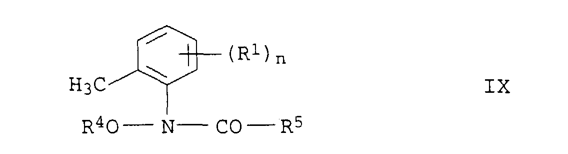 umwandelt, IX anschließend in das entsprechende Benzylhalogenid der Formel X 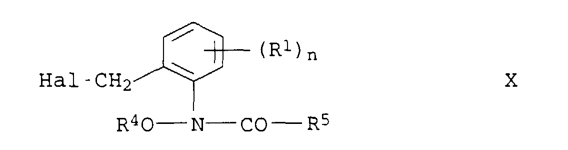 in der Hal für ein Halogenatom steht, überführt und X in Gegenwart einer Base mit einem 3-Hydroxytriazol der in Anspruch 2 definierten Formel III zu I umwandelt.

4. Verfahren zur Herstellung der Verbindungen I gemäß Anspruch 1, in denen R⁴ nicht Wasserstoff bedeutet und R⁵ für Methyl, Ethyl, Cyclopropyl oder Methoxy steht, dadurch gekennzeichnet, daß man eine entsprechende Verbindung der Formel I, in der R⁴ Wasserstoff bedeutet, mit einer Verbindung der in Anspruch 3 definierten Formel VIII umsetzt.

5. Verfahren zur Herstellung der Verbindungen der Formel I, in denen R⁵X für Methylamino steht, dadurch gekennzeichnet, daß man ein Benzylanilid der Formel IXa 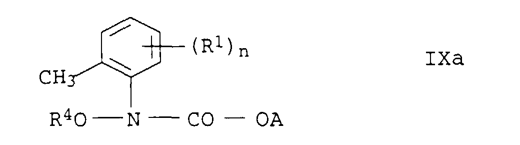 in der A für Alkyl oder Phenyl steht, in das entsprechende Benzylhalogenid der Formel Xa 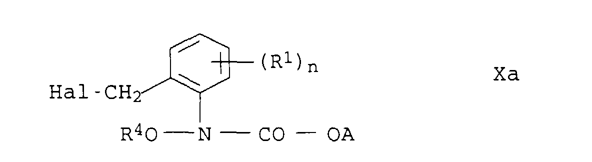 in der Hal für ein Halogenatom steht, überführt, Xa in Gegenwart einer Base mit einem 3-Hydroxytriazol der in Anspruch 2 definierten Formel III in eine Verbindung der Formel I.A 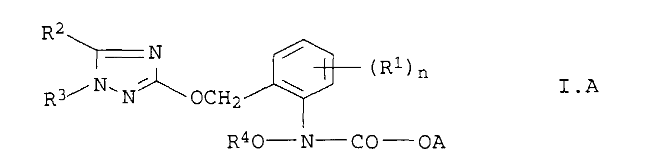 überführt und I.A anschließend mit Methylamin zu I umsetzt.

6. Zwischenprodukte der Formel XII 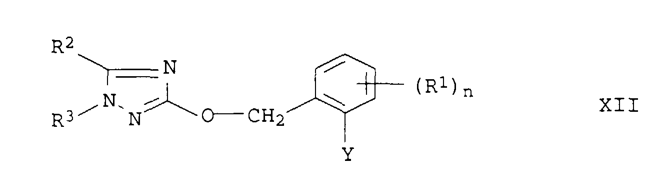 in der n und R¹, R² und R³ die in Anspruch 1 gegebene Bedeutung haben und Y für die folgenden Gruppen steht:
Y NO₂, NHOH oder NHOR⁴, wobei R⁴ die in Anspruch 1 gegebene Bedeutung hat.

7. Zwischenprodukte der allgemeinen Formel XIII 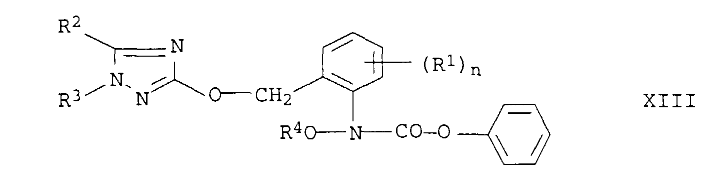 in der die Substituenten R¹, R², R³ und R⁴ sowie der Index n die in Anspruch 1 gegebene Bedeutung haben.

8. Zur Bekämpfung von Insekten, Spinnentieren, Nematoden oder Schadpilzen geeignetes Mittel, enthaltend einen festen oder flüssigen Trägerstoff und eine Verbindung der allgemeinen Formel I gemäß Anspruch 1.

9. Verfahren zur Bekämpfung von Schadpilzen, dadurch gekennzeichnet, daß man die Pilze oder die vor Pilzbefall zu schützenden Materialien, Pflanzen, Boden oder Saatgüter mit einer wirksamen Menge einer Verbindung der allgemeinen Formel I gemäß Anspruch 1 behandelt.

10. Verfahren zur Bekämpfung von Insekten, Spinnentieren und Nematoden, dadurch gekennzeichnet, daß man die Schädlinge oder die von ihnen zu schützenden Materialien, Pflanzen, Boden oder Saatgüter mit einer wirksamen Menge einer Verbindung der allgemeinen Formel I gemäß Anspruch 1 behandelt.

## Claims

1. A 2-[1',2',4'-triazol-3'-yloxymethylene]anilide of the formula I 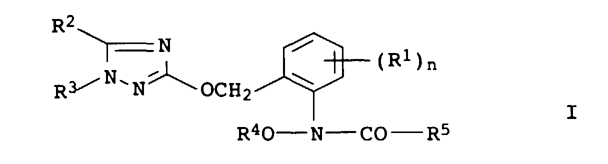 where the index and the substituents have the following meanings:
n is 0, 1, 2, 3 or 4, it being possible for the substituents R¹ to be different if n is greater than 1;
R¹ is halogen, C₁-C₄-alkyl, C₁-C₂-haloalkyl, C₁-C₄-alkoxy or C₁-C₂-haloalkoxy;
R² is hydrogen, nitro, cyano, halogen, C₁-C₄-alkyl, C₁-C₄-haloalkyl, C₁-C₄-alkoxy, C₁-C₄-alkylthio or C₁-C₄-alkoxycarbonyl;
R³ is C₁-C₄-alkyl or C₃-C₆-cycloalkyl
phenyl or benzyl, it being possible for the phenyl moiety to be partially or completely halogenated and/or
- to carry one to three of the following radicals: cyano, nitro, C₁-C₆-alkyl, C₁-C₄-haloalkyl, C₁-C₄-alkoxy, C₁-C₄-haloalkoxy, C₁-C₄-alkoxy-C₁-C₄-alkyl, C₃-C₆-cycloalkyl, C₁-C₄-alkylcarbonyl, C₁-C₄-alkoxycarbonyl, phenyl, phenoxy and phenyl-C₁-C₄-alkoxy,it being possible for the phenyl rings, for their part, to be partially or completely halogenated and/or to carry one to three of the following radicals: cyano, nitro, C₁-C₄-alkyl, C₁-C₂-haloalkyl, C₁-C₄-alkoxy, C₁-C₂-haloalkoxy, C₃-C₆-cycloalkyl, C₁-C₄-alkylcarbonyl or C₁-C₄-alkoxycarbonyl, and/or
- a group CR'=NOR", where R' is hydrogen or C₁-C₄-alkyl and R" is C₁-C₆-alkyl, and/or
- two adjacent C atoms of the phenyl ring via an oxy-C₁-C₃-alkoxy bridge or an oxy-C₁-C₃-haloalkoxy bridge,
pyridyl or pyrimidyl, it being possible for the heteroaromatic ring to be partially or completely halogenated and/or to carry one to three of the following radicals: cyano, nitro, C₁-C₄-alkyl, C₁-C₂-haloalkyl, C₁-C₄-alkoxy, C₁-C₂-haloalkoxy, C₃-C₆-cycloalkyl, C₁-C₄-alkylcarbonyl or C₁-C₄-alkoxycarbonyl;
R⁴ is hydrogen, C₁-C₄-alkyl or C₁-C₂-haloalkyl;
R⁵ is methyl, ethyl, cyclopropyl, methoxy or methylamino.

2. A process for preparing the compounds of the formula I as claimed in claim 1, where R⁴ is hydrogen and R⁵ is methyl, ethyl, cyclopropyl or methoxy, which comprises converting a benzyl derivative of the formula II 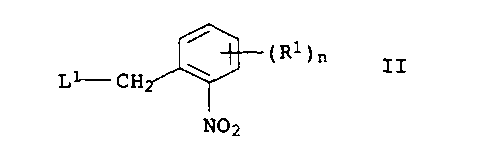 where L¹ is a nucleophilically replaceable group, in the presence of a base using a 3-hydroxytriazole of the formula III 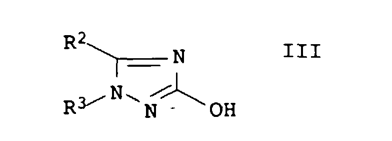 to the corresponding 2-[1',2',4'-triazol-3'-yloxymethylene]nitrobenzene of the formula IV 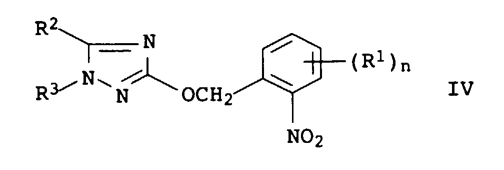 then reducing IV to the N-hydroxylaniline of the formula Va 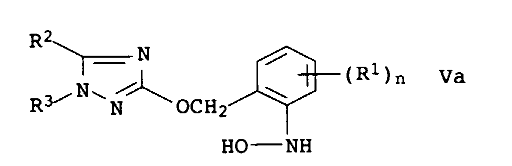 and converting Va to I using a carbonyl compound of the formula VI
L²―CO―R⁵ VI
where L² is a nucleophilically replaceable group.

3. A process for preparing the compounds of the formula I as claimed in claim 1, where R⁴ is not hydrogen and R⁵ is methyl, ethyl, cyclopropyl or methoxy, which comprises first reducing a benzyl derivative of the formula IIa 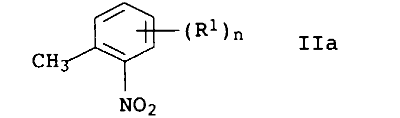 to the corresponding hydroxyaniline of the formula Vb 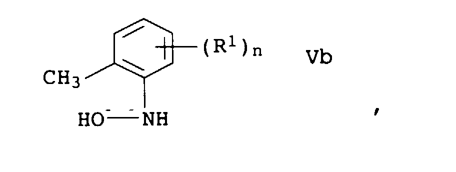 converting Vb using a carbonyl compound of the formula VI defined in claim 2 to the corresponding anilide of the formula VII 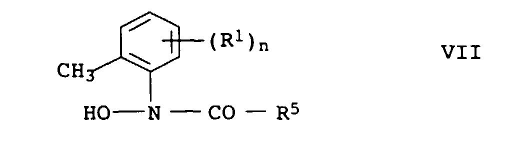 then converting VII using a compound VIII
L³-R⁴ VIII
where L³ is a nucleophilically replaceable group and R⁴ is not hydrogen, to the amide of the formula IX 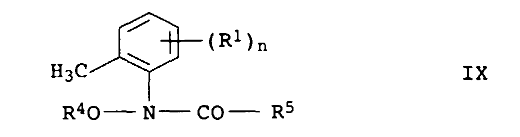 then converting IX to the corresponding benzyl halide of the formula X 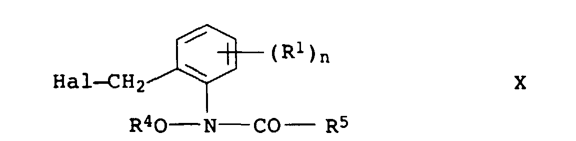 where Hal is a halogen atom, and converting X to I in the presence of a base using a 3-hydroxytriazole of the formula III defined in claim 2.

4. A process for preparing the compounds I as claimed in claim 1, where R⁴ is not hydrogen and R⁵ is methyl, ethyl, cyclopropyl or methoxy, which comprises reacting a corresponding compound of the formula I where R⁴ is hydrogen with a compound of the formula VIII defined in claim 3.

5. A process for preparing the compounds of the formula I where R⁵ is methylamino, which comprises converting a benzylanilide of the formula IXa 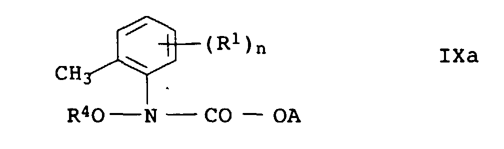 where A is alkyl or phenyl to the corresponding benzyl halide of the formula Xa 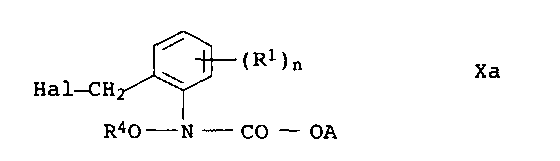 where Hal is a halogen atom, converting Xa in the presence of a base using a 3-hydroxytriazole of the formula III defined in claim 2 to a compound of the formula I.A 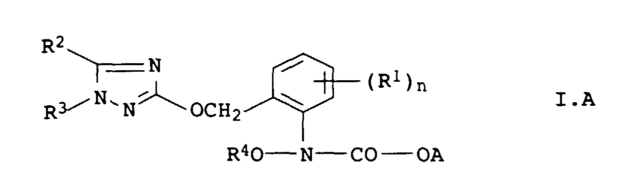 and then reacting I.A with methylamine
to give I.

6. An intermediate of the formula XII 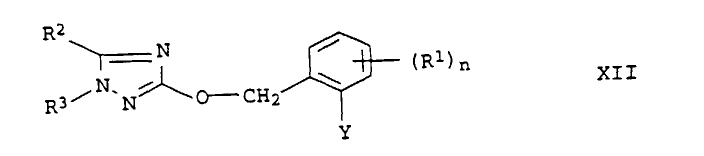 where n and R¹, R² and R³ have the meanings given in claim 1 and Y is the following groups:
Y is NO₂, NHOH or NHOR⁴, where R⁴ has the meaning given in claim 1.

7. An intermediate of the general formula XIII 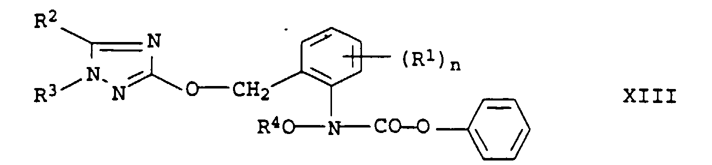 where the substituents R¹, R², R³ and R⁴ and the index n have the meanings given in claim 1.

8. A composition suitable for controlling insects, arachnids, nematodes or harmful fungi, containing a solid or liquid carrier and a compound of the general formula I as claimed in claim 1.

9. A method of controlling harmful fungi, which comprises treating the fungi or the materials, plants, soil or seed to be protected from fungal attack with an active amount of a compound of the general formula I as claimed in claim 1.

10. A method of controlling insects, arachnids and nematodes, which comprises treating the pests or the materials, plants, soil or seed to be protected from them with an active amount of a compound of the general formula I as claimed in claim 1.

## Revendications

1. 2-[1',2',4'-triazole-3'-yloxyméthylène]-anilides de formule I 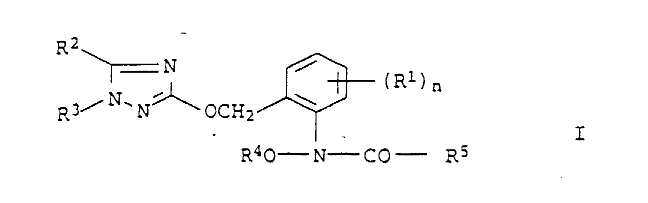 dans laquelle l'indice et les substituants ont- la signification suivante:
n 0, 1, 2, 3, ou 4, tandis que les substituants R¹ peuvent être différents lorsque n est supérieur à 1;
R¹ halogéno, alkyle en C₁-C₄, halogénoalkyle en C₁-C₂, alcoxy en C₁-C₄ ou halogénoalcoxy en C₁-C₂;
R² hydrogène, nitro, cyano, halogéno, alkyle en C₁-C₄, halogénoalkyle en C₁-C₄, alcoxy en C₁-C₄, alkylthio en C₁-C₄ ou alcoxy(C₁-C₄)carbonyle;
R³ alkyle en C₁-C₄ ou cycloalkyle en C₃-C₆;
phényle ou benzyle, tandis que la partie phényle peut être partiellement ou totalement halogénée et/ou peut porter
- un à trois des restes suivants: cyano, nitro, alkyle en C₁-C₆, halogénoalkyle en C₁-C₄, alcoxy en C₁-C₄, halogénoalcoxy en C₁-C₄, alcoxy(C₁-C₄)-alkyle(C₁-C₄), cycloalkyle en C₃-C₆, alkyl(C₁-C₄)carbonyle, alcoxy(C₁-C₄)carbonyle, phényle, phénoxy et phénylalcoxy(C₁-C₄), tandis que les cycles phényle peuvent à leur tour être partiellement ou totalement halogénés et/ou peuvent porter un à trois des substituants suivants: cyano, nitro, alkyle en C₁-C₄, halogénoalkyle en C₁-C₂, alcoxy en C₁-C₄, halogénoalcoxy en C₁-C₂, cycloalkyle en C₃-C₆, alkyl(C₁-C₄)carbonyle ou alcoxy(C₁-C₄)carbonyle, et/ou
- un groupe CR'=NOR", dans lequel R' représente l'hydrogène ou un alkyle en C₁-C₄ et R" désigne un alkyle en C₁-C₆,
et/ou
- deux atomes de carbone contigus du cycle phényle reliés par l'intermédiaire d'un pont oxy-alcoxy(C₁-C₃) ou d'un pont oxy-halogénoalcoxy(C₁-C₃),
pyridyle ou pyrimidyle, tandis que le cycle hétéroaromatique peut être partiellement ou totalement halogéné et/ou peut porter un à trois des restes suivants: cyano, nitro, alkyle en C₁-C₄, halogénoalkyle en C₁-C₂, alcoxy en C₁-C₄, halogénoalcoxy en C₁-C₂, cycloalkyle en C₃-C₆, alkyl(C₁-C₄)carbonyle ou alcoxy(C₁-C₄)carbonyle;
R⁴ hydrogène, alkyle en C₁-C₄ ou halogénoalkyle en C₁-C₂;
R⁵ méthyle, éthyle, cyclopropyle, méthoxy ou méthylamino.

2. Procédé pour la préparation de composés de formule I selon la revendication 1, dans lesquels R⁴ désigne l'hydrogène et R⁵ représente un groupe méthyle, éthyle, cyclopropyle ou méthoxy, caractérisé par le fait qu'on convertit un dérivé de benzyle de formule II, 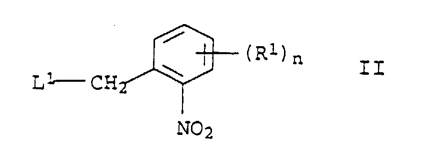 dans laquelle L¹ représente un groupe échangeable par voie nucléophile, en présence d'une base avec un 3-hydroxytriazole de formule III 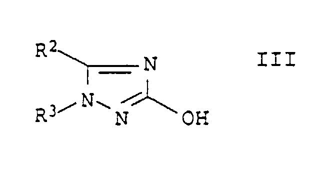 en le 2-[1',2',4'-triazole-3'-yloxyméthylène]-nitrobenzène correspondant de formule IV 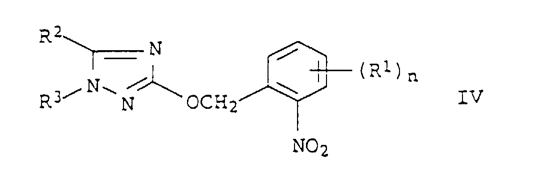 on réduit ensuite IV en la N-hydroxyaniline de formule Va 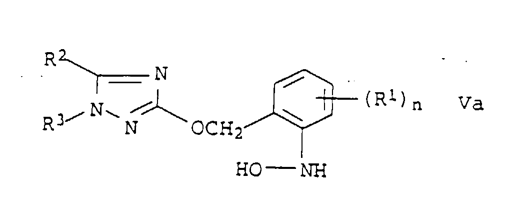 et on convertit Va en I avec un composé carbonylé de formule VI
L²―CO―R⁵ VI
dans laquelle L² représente un groupe échangeable par voie nucléophile.

3. Procédé pour la préparation de composés de formule I selon la revendication 1, dans lesquels R⁴ ne désigne pas l'hydrogène et R⁵ représente un groupe méthyle, éthyle, cyclopropyle ou méthoxy, caractérisé par le fait qu'on réduit d'abord un dérivé de benzyle de formule IIa 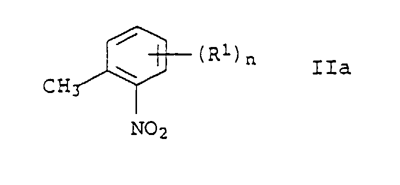 en l'hydroxyaniline correspondante de formule Vb 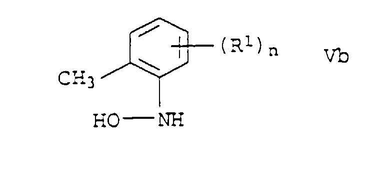 on convertit Vb avec un composé carbonylé ayant la formule VI définie dans la revendication 2 en l'anilide correspondant de formule VII 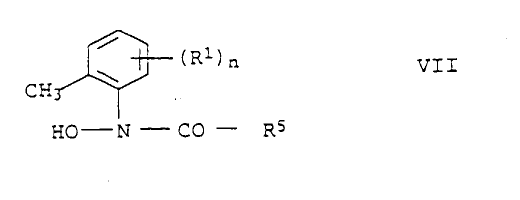 on transforme ensuite VII avec un composé VIII
L³-R⁴ VIII
dans lequel L³ représente un groupe échangeable par voie nucléophile et R⁴ ne désigne pas l'hydrogène, en l'amide de formule IX 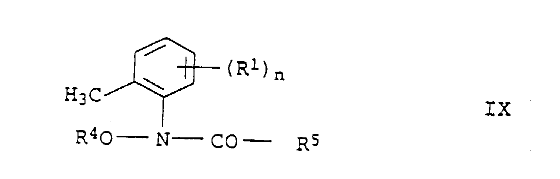 on convertit ensuite IX en l'halogénure de benzyle correspondant de formule X 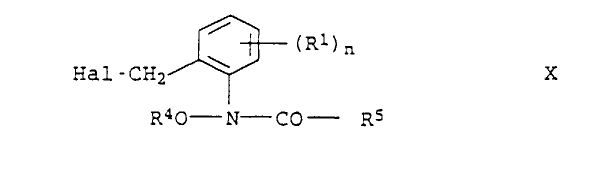 dans laquelle Hal désigne un atome d'halogène, et on transforme X en I en présence d'une base avec un 3-hydroxytriazole de formule III définie dans la revendication 2.

4. Procédé pour la préparation des composés I selon la revendication 1, dans lesquels R⁴ ne désigne pas l'hydrogène et R⁵ représente un groupe méthyle, éthyle, cyclopropyle ou méthoxy, caractérisé par le fait qu'on fait réagir un composé correspondant de formule I, dans laquelle R⁴ désigne l'hydrogène, avec un composé de formule VIII définie dans la revendication 3.

5. Procédé pour la préparation des composés de formule I, dans lesquels R⁵ désigne un groupe méthylamino, caractérisé par le fait qu'on convertit un benzylanilide de formule IXa 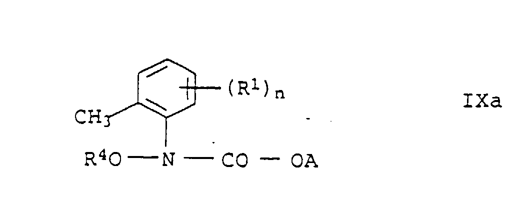 dans laquelle A désigne un groupe alkyle ou phényle, en l'halogénure de benzyle correspondant de formule Xa 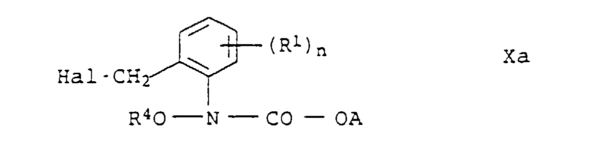 dans laquelle Hal désigne un atome d'halogène, on convertit Xa en présence d'une base avec un 3-hydroxytriazole de formule III définie dans la revendication 2, en un composé de formule I.A 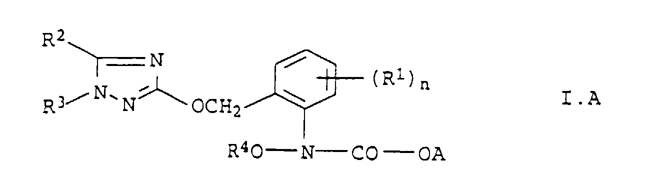 et on convertit ensuite I.A en I avec de la méthylamine.

6. Produits intermédiaires de formule XII 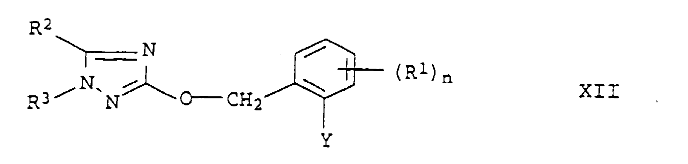 dans laquelle n et R¹, R² et R³ ont la signification indiquée dans la revendication 1 et Y représente les groupes suivants:
Y NO₂ , NHOH ou NHOR⁴, tandis que R⁴ a la signification indiquée dans la revendication 1.

7. Produits intermédiaires de formule générale XIII 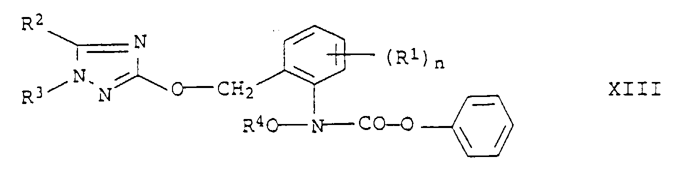 dans laquelle les substituants R¹, R², R³ et R⁴, ainsi que l'indice n, ont la signification indiquée dans la revendication 1.

8. Produit approprié pour la lutte contre les insectes, les arachnides, les nématodes ou les champignons nuisibles, contenant un support solide ou liquide et un composé de formule générale I selon la revendication 1.

9. Procédé pour la lutte contre les champignons nuisibles, caractérisé par le fait qu'on traite les champignons ou les matériaux, plantes, sols ou semences à protéger contre l'attaque par des champignons avec une quantité efficace d'un composé de formule générale I selon la revendication 1.

10. Procédé pour la lutte contre les insectes, les arachnides et les nématodes, caractérisé par le fait qu'on traite les parasites ou les matériaux, plantes, sols ou semences à protéger contre eux, avec une quantité efficace d'un composé de formule générale I selon la revendication 1.
